# EUROPEAN PATENT SPECIFICATION

(11) **EP 2 406 397 B1**
(45) Date of publication and mention of the grant of the patent: **17.07.2019**
(21) Application number: 10717217.3
(22) Date of filing: 01.03.2010
(51) Int. Cl.: C12Q 1/6886

(54) **METHODS USING AXL AS A BIOMARKER OF EPITHELIAL-TO-MESENCHYMAL TRANSITION**
VERFAHREN UNTER VERWENDUNG VON AXL ALS BIOMARKER FÜR DEN EPITHELIAL-MESENCHYMAL-ÜBERGANG
METHODES UTILISANT AXL COMME BIOMARQUEUR DE LA DIFFERENTIATION EPITHELIO-MESENCHYMATEUSE

(30) Priority: 13.03.2009 GB 0904418; 10.07.2009 GB 0912074
(43) Date of publication of application: 18.01.2012
(73) Proprietor: Bergen Teknologioverforing AS, Stedkode 709000 (NO)
(72) Inventor: LORENS, James, Bradley, N-5152 Bønes (NO); MICKLEM, David, Robert, N-5033 Bergen (NO); AKSLEN, Lars, N-5252 Soreidgrend (NO)
(74) Representative: Mewburn Ellis LLP
(86) International application number: PCT/IB2010/000516
(87) International publication number: WO 2010/103388

(56) References cited:
- WO-A1-2008/127707
- WO-A2-2004/008147
- WO-A2-2008/080134
- WO-A2-2008/086342
- WO-A2-2008/098139
- WO-A2-2009/007390
- US-A- 5 468 634
- MALARD VERONIQUE ET AL: "Global gene expression profiling in human lung cells exposed to cobalt" BMC GENOMICS, BIOMED CENTRAL, LONDON, GB LNKD- DOI:10.1186/1471-2164-8-147, vol. 8, no. 1, 6 June 2007 (2007-06-06), page 147, XP021027961 ISSN: 1471-2164
- TAI K-Y ET AL: "Axl promotes cell invasion by inducing MMP-9 activity through activation of NF-kappa B and Brg-1" ONCOGENE, vol. 27, no. 29, July 2008 (2008-07), pages 4044-4055, XP002598060 ISSN: 0950-9232
- ZHANG YI-XIANG ET AL: "AXL is a potential target for therapeutic intervention in breast cancer progression" CANCER RESEARCH / AMERICAN ASSOCIATION FOR CANCER RESEARCH, AACR, PHILADELPHIA, PA LNKD- DOI:10.1158/0008-5472.CAN-07-2661, vol. 68, no. 6, 15 March 2008 (2008-03-15) , pages 1905-1915, XP002505662 ISSN: 1538-7445
- VAJKOCZY PETER ET AL: "Dominant-negative inhibition of the Axl receptor tyrosine kinase suppresses brain tumor cell growth and invasion and prolongs survival" PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.0510923103, vol. 103, no. 15, 11 April 2006 (2006-04-11), pages 5799-5804, XP002505666 ISSN: 0027-8424
- GJERDRUM CHRISTINE ET AL: "Axl is an essential epithelial-to-mesenchymal transition-induced regulator of breast cancer metastasis and patient survival." PROCEEDINGS OF THE NATIONAL ACADEMY OF SCIENCES OF THE UNITED STATES OF AMERICA 19 JAN 2010 LNKD- PUBMED:20080645, vol. 107, no. 3, 19 January 2010 (2010-01-19), pages 1124-1129, XP002598061 ISSN: 1091-6490
- HOLLAND SACHA J ET AL: "R428, a Selective Small Molecule Inhibitor of Axl Kinase, Blocks Tumor Spread and Prolongs Survival in Models of Metastatic Breast Cancer" CANCER RESEARCH, vol. 70, no. 4, February 2010 (2010-02), pages 1544-1554, XP002598062 ISSN: 0008-5472
- GJERDRUM CHRISTINE ET AL: "The receptor tyrosine kinase Axl controls breast carcinoma cell invasiveness and tumor formation by regulating E-cadherin", CLINICAL & EXPERIMENTAL METASTASIS, vol. 26, no. 7, October 2009 (2009-10), page 863, & JOINT CONFERENCE OF THE METASTASIS-RESEARCH-SOCIETY/AMERICAN-ASSOC IATION-FOR-CANCER-RESEARCH; VANCOUVER, CANADA; AUGUST 03 -07, 2008 ISSN: 0262-0898
- THIERY J P: "Epithelial-Mesenchymal Transitions in Tumor Progression", NATURE REVIEWS. CANCER, NATUR PUBLISHING GROUP, LONDON, GB, vol. 2, 1 June 2002 (2002-06-01), pages 442-454, XP002990279, ISSN: 1474-175X, DOI: 10.1038/NRC822
- SHARON BARR ET AL: "Bypassing cellular EGF receptor dependence through epithelial-to-mesenchymal-like transitions", CLINICAL & EXPERIMENTAL METASTASIS ; OFFICIAL JOURNAL OF THEMETASTASIS RESEARCH SOCIETY, KLUWER ACADEMIC PUBLISHERS, DO, vol. 25, no. 6, 31 January 2008 (2008-01-31), pages 685-693, XP019601763, ISSN: 1573-7276

## Description

The present disclosure relates to a biomarker and diagnostic/prognostic method for detecting the occurrence of epithelial-to-mesenchymal transition (EMT). More specifically, the disclosure relates to diagnostic, prognostic and therapeutic methods involving the expression and/or activity of Axl.

### BACKGROUND

Axl is a member of the receptor tyrosine kinase sub-family. Although similar to other receptor tyrosine kinases, the Axl protein represents a unique structure of the extracellular region that juxtaposes IgL and FNIII repeats, and has an intracellular region containing an intracellular domain, part of which is the kinase domain. Axl transduces signals from the extracellular matrix into the cytoplasm by binding growth factors like vitamin K-dependent protein growth-arrest-specific gene 6 (Gas6). The extracellular domain of Axl can be cleaved and a soluble extracellular domain of 65 kDa can be released. Cleavage enhances receptor turnover and generates a partially activated kinase (O'Bryan JP, et al (1995) J Biol Chem. 270 (2): 551-557). However, the function of the cleaved domain is unknown.

Structural information relating to the human Axl gene and gene product is described in WO 03/068983. The following patent publications also relate to Axl or other tyrosine kinase receptors: US 5,468,634; US 6,087,144; US 5,538, 861; US 5,968, 508; US 6,211,142; US 6,235,769; WO 99/49894; WO 00/76309; WO 01/16181 and WO 01/32926.

Axl is involved in the stimulation of cell proliferation. Specifically, Axl is a chronic myelogenous leukemia-associated oncogene, that is also associated with colon cancer and melanoma. It is in close vicinity to the bcl3 oncogene which is at 19q13.1-q13.2. The Axl gene is evolutionarily conserved among vertebrate species, and is expressed during development in the mesenchyme.

Upon interaction with the Gas6 ligand, Axl becomes autophosphorylated, and a cascade of signal transduction events takes place. PI3K, AKT, src, Bad, 14-3-3, PLC, ERK, S6K (mitogen-regulated kinase) and STAT are each known to be involved in this cascade. Gas6 has a region rich with y-carboxyglutamic acid (GLA domain) that allows for Ca++-dependent binding to membrane phospholipids. Gas6 is a weak mitogen and has an anti-apoptotic effect in NIH3T3 fibroblasts subjected to stress by TNF-induced cytotoxicity, or growth factor withdrawal. In NIH3T3 the binding of Gas6 to Axl results in activation of PI3K, AKT, src and Bad.

Studies have shown that Axl plays a number of different roles in tumour formation. Axl is a key regulator of angiogenic behaviours including endothelial cell migration, proliferation and tube formation. Axl is also required for human breast carcinoma cells to form a tumour *in vivo,* indicating that Axl regulates processes that are vital for both neovascularisation and tumorigenesis (Holland S. et al, Cancer Res 2005; 65 (20), Oct 15, 2005).

The activity of Axl receptor tyrosine kinase is positively correlated with tumour metastasis. More specifically, studies have shown that Axl enhances expression of MMP-9, which is required for Axl-mediated invasion. Axl promotes cell invasion by inducing MMP-9 acitivity through activation of NF-Bκ and Brg-1 (Tai, K-Y et al, Oncogene (2008), 27, 4044-4055).

Axl is overexpressed in human glioma cells and can be used to predict poor prognosis in patients with Glioblastoma Multiforme (GBM) (Vajkoczy P. et al, PNAS, April 11, 2006, vol 103, no. 15, 5799-5804; Hutterer M. et al, Clinical Cancer Res 2008; 14 (1) Jan 1, 2008;). Axl is also relatively overexpressed in highly invasive lung cancer cell lines compared to their minimally invasive counterparts (Shieh, Y-S et al, Neoplasia, vol 7, no. 12, Dec 2005, 1058-1064). Axl is therefore believed to play an important role in tumour invasion and progression.

Likewise, Axl is expressed in highly invasive breast cancer cells, but not in breast cancer cells of low invasivity. More specifically, inhibition of Axl signalling (by dominant-negative Axl mutant, an antibody against the extracellular domain of Axl, or by short hairpin RNA knockdown of Axl) decreased the motility and invasivity of highly invasive breast cancer cells. Small molecule Axl inhibitors interfered with motility and invasivity of breast cancer cells. Thus, Axl is understood to be a critical element in the signalling network that governs the motility/invasivity of breast cancer cells (Zhang, Y-X et al, Cancer Res 2008; 68 (6), March 15, 2008).

In mesangial cells, Gas6 was found to have a mitogenic effect, indicative of a possible role in the progression of glomerulosclerosis. Evidence has suggested that the Gas6/Axl pathway also plays a role in glomerulonephritis (Yanagita M. at al, The Journal of Clinical Investigation, 2002, 110 (2) 239-246). Further studies have shown that Gas6 promotes the survival of endothelial cells in a model for arterial injury. Angiotensin II, via its AT1 receptor, was shown to increase Axl mRNA and protein receptor in vascular smooth muscle cells (Melaragno M. G. et al, Circ Res., 1998, 83 (7): 697- 704).

Axl has also been shown to be involved in cellular adhesion, cell proliferation and regulation of homeostasis in the immune system (Lu Q., 2001) Science 293 (5528): 306-311). Following Axl activation, the following phenomena have been observed: inhibition of apoptosis, increase in "normal" cell (non-transformed) survival of fibroblasts and endothelial cells, migration of Vascular Smooth Muscle Cell (VSMC) (inactivation of the Axl kinase blocks migration), enhancement of neointima formation in blood vessel wall (Melaragno M.G. et al, Trends Cardiovasc Med., 1999, (Review) 9 (8): 250-253) and involvement in lesion formation and the progression of atherosclerosis.

Described herein are new diagnostic, prognostic and therapeutic applications involving Axl. In particular, the disclosure provides methods for detecting the occurrence of epithelial-to-mesenchymal transition (EMT), which have therapeutic implications in the treatment of cancer, more specifically, metastatic and drug resistant cancers.

### SUMMARY OF DISCLOSURE

The present invention is as defined in the claims.

Disclosed herein is the use of Axl as a biomarker for detecting the occurrence of epithelial-to-mesenchymal transition (EMT) in a subject.

The present disclosure is based on the finding that Axl expression is correlated to the occurrence of epithelial-to-mesenchymal transition (EMT). To our knowledge, this represents the first demonstration of such a correlation. Advantageously, this finding opens up exciting new opportunities for providing diagnostic, prognostic and therapeutic methods in the field of cancer, more particularly, metastatic and drug resistant cancers.

WO 2008/080134 relates to "Diaminothiazoles and pharmaceutical compositions containing them" and "using the diaminothiazoles in treating diseases or conditions associated with Axl activity".

WO 2008/098139 relates to "novel inhibitors of the Axl receptor tyrosine kinase (RTK) and methods of using such inhibitors in a variety of therapeutic approaches in the areas of cancer therapy and anti-thrombosis (anti-clotting) therapy".

WO 2009/007390 relates to "pyrazine derivatives" and "processes for their preparation, pharmaceutical compositions containing them and their use in the manufacture of a medicament for use in the treatment of cell proliferative disorders".

WO 2004/008147 relates to "diagnostic and therapeutic methods in the field of malignant disorders".

US 5468634 relates to "Isolated DNA sequences encoding a mammalian axl receptor which exhibits axl oncogene activity" and "antibodies which specifically bind the axl receptor".

Malard et al, BMC Genomics, 2007,8:147 relates to "Global gene expression profiling in human lung cells exposed to cobalt".

Tai et al, Oncogene, 2008, 27(29:4044-55 describes data which "provide a mechanism for Axl-mediated tumor invasion and establish a functional link between the Axl and MMP-9 signaling pathways".

Zhang et al, Cancer Res., 2008, 68(6):1905-15 relates to "the identification of experimental anti-AXL small molecular inhibitors that represent lead substances for the development of antimetastatic breast cancer therapy".

Vajkoczy et al, Proc Natl Acad Sci USA, 2006, 103(15):5799-804 relates to data identifying "the receptor tyrosine kinase Axl as a mediator of glioma growth and invasion".

Gjerdum et al, Proc Natl Acad Sci USA, 2010, 107(3):1124-9 relates to data suggesting "Axl represents a downstream effector of the tumor cell EMT that is required for breast cancer metastasis".

Holland et al, Cancer Res, 2010, 70(4):1544-54 relates to data showing "that Axl signaling regulates breast cancer metastasis at multiple levels in tumor cells and tumor stromal cells and that selective Axl blockade confers therapeutic value in prolonging survival of animals bearing metastatic tumors".

Gjerdum et al, Clin Exp Metastasis, 2009, 26(7):863 relates to data showing that "Axl regulate E-cadherin expression in MDA-MB-231 cells causing the cells to become more invasive".

Thiery et al, Nat Rev Cancer, 2002, 2(6):442-54 relates to "Epithelial-mesenchymal transitions in tumour progression".

Barr et al, Clin Exp Metastasis, 2008, 25(6):685-693 relates to "Bypassing cellular EGF receptor dependence through epithelial-to-mesenchymal-like transitions".

The present inventors have demonstrated a previously unrecognized role for the receptor tyrosine kinase Axl as an essential EMT-induced effector in the invasion-metastasis cascade. The results show that EMT program activation leads to Axl upregulation that is essential for invasiveness and spontaneous metastasis of malignant breast carcinoma cells and drug resistance phenotype, Axl expression correlates strongly with breast cancer patient mortality from interval mammography-detected tumors and clinically identified breast tumours, suggesting a link between Axl activation and the development of metastatic disease.

Also disclosed herein is a method for detecting the occurrence of epithelial-to-mesenchymal transition (EMT) in a sample, said method comprising the steps of:
(i) isolating a sample from a cell, group of cells, an animal model or human;
(ii) determining the expression of Axl in said sample as compared to a control sample, wherein upregulation of Axl expression relative to the control sample is indicative of the occurrence of epithelial-to-mesenchymal transition (EMT).

Advantageously, determining the expression of Axl provides a "permanent" marker for detecting the occurrence of an EMT event, which in itself is transient in nature. Thus, the detection of Axl expression provides a unique and permanent indication of whether an EMT event has taken place.

Studies by the Applicant have shown that this is due to the fact that EMT-related activation establishes an autocrine Axl-Gas6 signalling loop that is advantageous to malignant cells. Axl may also be activated via paracrine mechanisms.

Detection of tumor cells that have undergone EMT is complicated by the fact that current markers (eg. vimentin, N-Cadherin, lack of E-cadherin) are based on mesenchymal cytoskeletal and junctional proteins that are present in normal stromal cells. Distinguishing tumor cells from surrounding stroma cells is difficult. Axl expression is more likely to be restricted to tumor cells in solid tumors providing a distinguishing characteristic to malignant tumor cells.

Reversibility of EMT is important for metastasis formation at distant sites. Axl expression can be used to detect metastasis.

Also disclosed herein is a method of diagnosing metastatic cancer in a subject by detecting the occurrence of epithelial-to-mesenchymal transition (EMT), said method comprising determining the level of an Axl receptor polypeptide in a sample from the subject, wherein a higher level of the polypeptide compared to the level in a subject free of metastatic cancer is indicative of the occurrence of epithelial-to-mesenchymal transition (EMT).

Also disclosed is the use of Axl or a gene encoding Axl in monitoring the activity of an agent capable of inhibiting or reversing epithelial-to-mesenchymal transition (EMT).

Also disclosed is a method for identifying an agent capable of inhibiting or reversing epithelial-to-mesenchymal transition (EMT), said method comprising administering said agent to a cell, group of cells, animal model or human and monitoring the activity and/or or expression of Axl.

Also disclosed herein is a method for detecting the ability of an agent to inhibit or reverse epithelial-to-mesenchymal transition (EMT), said method comprising:
(i) administering the agent to a cell, group of cells, an animal model or human;
(ii) measuring Axl expression in samples derived from the treated and the untreated cells, animal or human; and
(iii) detecting an increase or a decrease in the expression or activity of Axl in the treated sample as compared to the untreated sample as an indication of the ability to inhibit or reverse epithelial-to-mesenchymal transition (EMT).

Also disclosed is a method of monitoring the activity of an Axl inhibitor, said method comprising detecting the occurrence of epithelial-to-mesenchymal transition (EMT) by:
(i) administering said Axl inhibitor to a cell, group of cells, an animal model or human; and
(ii) measuring Axl expression in samples derived from the treated and the untreated cells, animal or human; and
(iii) detecting an increase or a decrease in the expression or activity of Axl in the treated sample as compared to the untreated sample as an indication of Axl inhibitory activity.

Also disclosed is a method for identifying an agent capable of inhibiting or reversing epithelial-to-mesenchymal transition (EMT), said method comprising the steps of:
(i) contacting the agent with Axl receptor or cells expressing the Axl receptor;
(ii) measuring the Axl receptor activity in the presence of the agent; and
(iii) comparing the activity measured in step (ii) to that measured under controlled conditions, wherein a decrease identifies the agent as being capable of inhibiting or reversing epithelial-to-mesenchymal transition (EMT).

Also disclosed is a method for identifying an agent capable of inhibiting or reversing epithelial-to-mesenchymal transition (EMT) by screening a plurality of agents, said method comprising the steps of:
(i) contacting the plurality of agents with the Axl receptor or cells expressing the Axl receptor;
(ii) measuring the Axl receptor activity in the presence of the plurality of agents;
(iii) comparing the activity measured in step (ii) to that measured under controlled conditions, wherein a decrease identifies the plurality of agents as being capable of inhibiting or reversing epithelial-to-mesenchymal transition (EMT); and
(iv) separately determining which agent or agents present in the plurality inhibit or reverse epithelial-to-mesenchymal transition (EMT).
the use of an agent identified according to the method of the disclosure in the preparation of a medicament for the treatment of metastatic cancer is also provided.

Also disclosed is a pharmaceutical composition comprising an agent identified according to the method of the disclosure admixed with a pharmaceutically acceptable diluent, excipient or carrier.

Also disclosed is a process of preparing a composition which comprises:
(i) identifying an agent capable of inhibiting or reversing epithelial-to-mesenchymal transition (EMT) using the method according to the disclosure; and
(ii) admixing said agent with a pharmaceutically acceptable diluent, carrier or excipient.

Also disclosed herein is a method for inhibiting or reversing epithelial-to-mesenchymal transition (EMT) in a subject in need thereof, said method comprising administering an Axl inhibitor to said subject.

Also disclosed is a method for treating metastatic cancer in a subject in need thereof, said method comprising inhibiting or reversing epithelial-to-mesenchymal transition (EMT) by administering to said subject an Axl inhibitor.

Also disclosed is the use of an Axl inhibitor in the preparation of a medicament for inhibiting or reversing epithelial-to-mesenchymal transition (EMT).

Also disclosed herein is the use of an Axl inhibitor in the preparation of a medicament for treating metastatic cancer by inhibiting or reversing epithelial-to-mesenchymal transition (EMT).

Also disclosed is a kit for assessing the ability of an agent to inhibit or reverse epithelial-to-mesenchymal transition (EMT), said kit comprising anti-Axl antibodies, a nucleic acid probe for Axl or a QPCR primer for Axl.

Also disclosed is the use of a kit as defined above in a method according to the disclosure.

### DETAILED DESCRIPTION

Metastasis underlies the majority of cancer-related deaths. Hence, furthering the understanding of the molecular mechanisms that enable tumour cell dissemination is a vital health issue. Epithelial-to-mesenchymal transitions (EMT) endow carcinoma cells with enhanced migratory and survival attributes that facilitate malignant progression. Characterization of EMT effectors is likely to yield new insights into metastasis and novel avenues for treatment. The Applicant has shown that the presence of the receptor tyrosine kinase Axl in mammography-detected primary breast cancers independently predicts strongly reduced overall patient survival, and matched patient metastasis lesions show enhanced Axl expression. The Applicant has also demonstrated that Axl is strongly induced by epithelial-to-mesenchymal transition in pre-malignant mammary epithelial cells that establishes an autocrine signalling loop with its ligand, Gas6. Using epi-allelic RNA interference analysis in metastatic breast cancer cells, the Applicant delineated a distinct threshold of Axl expression for mesenchymal-like in vitro cell invasiveness, and to form tumours in foreign and tissue engineered microenvironments *in vivo.* Importantly, Axl knockdown completely prevented the spread of highly metastatic breast carcinoma cells from the mammary gland to lymph nodes and several major organs, and increased overall survival, in two different optical imaging-based experimental breast cancer models. Thus, Axl represents a novel downstream effector of tumour cell EMT that is required for breast cancer metastasis. The detection and targeted treatment of Axl-expressing tumours represents an important new therapeutic strategy for breast cancer.

### Role of Axl in EMT and Metastasis

The acquisition of mesenchymal cellular characteristics endows epithelial cancer cells with the unicellular invasive cell motility associated with metastasis (Theirry, 2002 Weinberg, 2007).

As mentioned above, the present inventors have demonstrated that Axl is an essential EMT-induced effector in the invasion-metastasis cascade. The results show that EMT program activation leads to Axl upregulation that is essential for invasiveness and spontaneous metastasis of malignant breast carcinoma cells. Axl expression correlates strongly with breast cancer patient mortality from interval mammography-detected tumors, suggesting a link between Axl activation and the development of metastatic disease.

Axl was originally identified as a key regulator of invasive cell migration in a functional genetic screen (Holland *et al* 2005). We demonstrate here that Axl expression in malignant breast cancer cells is required for invasiveness in three-dimensional matrices in response to different chemotactic inducers (serum, SDF-1). In contrast, we observed little effect of Axl knockdown in plate-based 2D assays (proliferation, scratch) or on cell adhesion. Axl inhibition also inhibits glioma and lung carcinoma cell migration without affecting proliferation (Angelillo-Scherrer *et al.,* 2005; Shieh *et al.,* 2005). Hence a common theme is that Axl signaling is essential for mesenchymal migratory phenotype in malignant tumor cells.

Congruent with this we demonstrate that Axl represents a novel marker of EMT induced by several transcription factors including Twist, Snail, Slug and ZEP2. Expression of these transcription factors in epithelial cells elicits a normal developmental program that transiently upregulates mesenchymal characteristics in epithelial cells. Pre-malignant epithelial cells are thought to active EMT via contextual signals such as TGFbeta elaborated by local stroma cells (Weinberg, 2007). This process is dynamic *in vivo* as EMT-associated marker expression such as E-cadherin varies greatly in tumors. Indeed, Axl is an independent prognosticator in our studies and does not correlate with changes in E-cadherin. This lack of clinical evidence for EMT is well documented and debated. The Applicant has shown that Axl expression represents a more durable EMT-induced change.
In order to determine whether Axl is essential for metastasis from the mammary microenvironment, we implanted a highly metastatic (*in vivo* passaged) breast carcinoma cell line (MDA-231-DH2LN) into mammary glands and monitored spread by *in vivo* optical imaging of luciferase bioluminescence. Temporal whole body optical imaging revealed extensive MDA-231-DH2LN spread to lymph nodes, lungs, ovaries and kidneys within 28 days of implantation in all control mice. Spontaneous lymph node metastases were detected initially at 4 weeks in all animals. Organ metastases were detected throughout the 9-week follow up. At sacrifice, excised organs were scanned individually and shown to contain metastases, later confirmed by histology. In contrast, no metastases were detected by bioluminescence or histology of organs from mice bearing Axl knockdown cells (MDA-231-DHLN-AxlshRNA). This strong inhibition of spread from orthotopic mammary site shows that Axl is essential for metastasis.

Together, our results indicate that detection and targeted treatment of Axl-expressing mammary tumors represents an important new strategy for breast cancer therapeutic development.

### Diagnostic Tool

Disclosed herein is a diagnostic tool for detecting the occurrence of epithelial-to-mesenchymal transition (EMT).

Thus, the dislcosure provides the use of Axl as a biomarker for detecting the occurrence of epithelial-to-mesenchymal transition (EMT) in a subject.

in one preferred case, Axl is a biomarker for detecting the induction of epithelial-to-mesenchymal transition (EMT).

Metastasis to distant sites is the most common cause of death from solid tumors (Gupta 2006, Spom 1996). To accomplish this, tumor cells discard epithelial restraints, redefine junctional complexes and acquire invasive motility to break across the basement membrane border. These metastatic cells then intravasate into the lymphatic and hematogenous circulation, disseminating to distant sites in the body. A few of these metastatic cells succeed in extravasating through the capillary wall and in rare cases colonize the foreign tissue stroma (Weinberg *et al*). This malignant process is facilitated by an epithelial-to-mesenchymal transition (EMT), a developmental program where epithelial cells transiently assume a mesenchymal phenotype during gastrulation and organogenesis, allowing single cell invasive movement away from the epithelial layer (Hall, 1985; Thierry, 2002). The EMT program is initiated by contextual activation of morphogen signaling pathways that induce the expression of transcriptional regulators, including Twist, Snail, Slug and Zeb2, which alter the expression of junctional complex proteins (Thiery and SLeeman 2006). The EMT gene expression profile reflects the phenotypic shift, repression of E-cadherin and cytokeratins with induction of vimentin and N-cadherin (Weinberg *et al* 2007).

Also disclosed is the use of Axl as a biomarker for detecting and monitoring malignancy.

Also disclosed is the use of Axl as a biomarker for detecting tumour metastasis. Preferably, the tumour is a carcinoma, more preferably breast cancer.

The disclosure provides a diagnostic method for determining whether a subject would be a suitable candidate to receive treatment with an Axl inhibitor. For example, if Axl expression is shown to be upregulated, this can be used as a guide to treatment options and performance, i.e. a prognostic in personalised medicine applications, to select subjects that are likely to be susceptible to treatment with an Axl inhibitor. For example, if Axl expression is shown to be upregulated in a primary tumor, this can be used to infer an increased probability of metastasis. This information can be used as a guide to treatment options, i.e. a prognostic in personalised medicine applications, to select subjects that are likely to need more aggressive anti-cancer surgical, chemotherapeutic or radiotherapeutic treatment such as radical mastectomy.

Thus, also disclosed herein is a method for determining whether a subject will be susceptible to treatment with an Axl inhibitor, said method comprising the steps of:
(i) isolating a sample from a cell, group of cells, an animal model or human;
(ii) determining the expression of Axl in said sample as compared to a control sample, wherein upregulation of Axl expression relative to the control sample is indicative of susceptibility to treatment with an Axl inhibitor.

The term "marker" or "biomarker" is used herein to refer to a gene or protein whose expression in a sample derived from a cell or mammal is altered or modulated, for example, up or down regulated, when epithelial-to-mesenchymal transition (EMT) takes place. Where the biomarker is a protein, modulation or alteration of expression encompasses modulation through different post translational modifications.

Post translational modifications are covalent processing events that change the properties of a protein by proteolytic cleavage or by addition of a modifying group to one or more amino acids. Common post translational modifications include phosphorylation, acetylation, methylation, acylation, glycosylation, GPI anchor, ubiquitination and so forth. A review of such modifications and methods for detection may be found in Mann et al. Nature Biotechnology March 2003, Vol. 21, pages 255-261.

In one preferred case, upregulation of Axl is indicative of the occurrence of epithelial-to-mesenchymal transition (EMT).

The disclosure also provides a method for detecting the occurrence of epithelial-to-mesenchymal transition (EMT) in a sample, said method comprising the steps of:
(i) isolating a sample from a cell, group of cells, an animal model or human;
(ii) determining the expression of Axl in said sample as compared to a control sample, wherein upregulation of Axl expression relative to the control sample is indicative of the occurrence of epithelial-to-mesenchymal transition (EMT).

Also disclosed herein is a method of diagnosing metastatic cancer in a subject by detecting the occurrence of epithelial-to-mesenchymal transition (EMT), said method comprising determining the level of an Axl receptor polypeptide in a sample from the subject, wherein a higher level of the polypeptide compared to the level in a subject free of metastatic cancer is indicative of the occurrence of epithelial-to-mesenchymal transition (EMT).

In particular, cancers of interest include any carcinoma, more preferably breast, lung, gastric, head and neck, colorectal, renal, pancreatic, uterine, hepatic, bladder, endometrial and prostate cancers and leukemias. More preferably, the cancer is metastatic breast cancer.

Preferably, the expression of the Axl gene, or the level of Axl receptor polypeptide, is measured using an anti-Axl antibody or affinity agent.

### Diagnostic for new therapeutic agents

The present disclosure also provides a diagnostic assay for identifying agents that are capable of inhibiting or reversing epithelial-to-mesenchymal transition (EMT), thereby having potential therapeutic applications in the treatment of proliferative disorders such as cancer.

Thus, disclose herein is the use of Axl or a gene encoding Axl in monitoring the activity of an agent capable of inhibiting or reversing epithelial-to-mesenchymal transition (EMT).

In one preferred case the presence of Axl is monitored after administration of the agent capable of inhibiting or reversing epithelial-to-mesenchymal transition (EMT) to a cell, group of cells, an animal model or human.

Also disclosed is a method for identifying an agent capable of inhibiting or reversing epithelial-to-mesenchymal transition (EMT), said method comprising administering said agent to a cell, group of cells, animal model or human and monitoring the activity and/or or expression of Axl.

In one preferred case, the method comprises administering said agent to a cell, group of cells, animal model or human and detecting altered expression of Axl in said treated sample as compared to an untreated control sample.

By "altered expression" is meant an increase, decrease or otherwise modified level or pattern of expression in a sample derived from a treated cell when compared to an untreated, control sample.

The term "expression" refers to the transcription of a gene's DNA template to produce the corresponding mRNA and translation of this mRNA to produce the corresponding gene product (i.e., a peptide, polypeptide, or protein) as well as the "expression" of a protein in one or more forms that may have been modified post translation.

Detection of altered expression including gene expression may be performed by any one of the methods known in the art, particularly by microarray analysis, Western blotting or by PCR techniques such as QPCR. Altered expression may also be detected by analysing protein content of samples using methods such as ELISA, PET or SELDI-TOF MS as described herein and using further analytical techniques such as 2Dgel electrophoresis. Techniques such as this can be particularly useful for detecting altered expression in the form of alternative post translationally modified forms of a protein.

Also disclosed is a method for detecting the ability of an agent to inhibit or reverse epithelial-to-mesenchymal transition (EMT), said method comprising:
(i) administering the agent to a cell, group of cells, an animal model or human; and
(ii) measuring Axl expression in samples derived from the treated and the untreated cells, animal or human; and
(iii) detecting an increase or a decrease in the expression of Axl in the treated sample as compared to the untreated sample as an indication of the ability to inhibit or reverse epithelial-to-mesenchymal transition (EMT).

The inhibition may be at any level (e.g. at the gene expression level or the protein level).

Also disclosed herein is a method of monitoring the activity of an Axl inhibitor, said method comprising detecting the occurrence of epithelial-to-mesenchymal transition (EMT) by;
(i) administering said Axl inhibitor to a cell, group of cells, an animal model or human;
(ii) measuring Axl expression in samples derived from the treated and the untreated cells, animal or human; and
(iii) detecting an increase or a decrease in the expression or activity of Axl in the treated sample as compared to the untreated sample as an indication of Axl inhibitory activity.
preferably the sample is analysed by protein analysis, more preferably by ELISA, PET, flow cytometry, SELDI-TOF MS or 2-D PAGE.

As used herein, a sample derived from a treated or untreated cell can be a lysate, extract or nucleic acid sample derived from a group of cells which can be from tissue culture or animal or human. For protein analysis, a sample can be a tissue culture supernatant. A cell can be isolated from an individual (e.g. whole cells from a blood, serum or plasma sample) or can be part of a tissue sample such as a biopsy.

Preferably, the group of cells is a cell culture.

Preferred cell types are selected from colonic tumour cell lines such as HT29, lung tumour cell lines such as A549, renal tumour cell lines such as A498, bladder tumour cell lines such as HT13, breast tumour cell lines such as MDA-MB-231, endometrial tumour cell lines such as AN3CA, uterine tumour cell lines such as MESSA DH6 uterine sarcoma cells, hepatic tumour cell lines such as Hep2G, prostate tumour cell lines such as DU145, T cell tumour cell lines such as Cem T cell, pancreatic tumour cell lines such as MiaPaCa2. Alternatively, the cells may be in the form of a histological sample of a tumor biopsy (such as a sample taken by laser capture microsurgery). Suitable methods for detecting gene expression in biopsy samples include using FISH or immunohistochemistry techniques using antibodies that recognise the genes identified herein as well as methods for analysing the protein composition of samples.

In another alternative, the cells may be blood cell cultures such as PBMCs. As used herein, the term "PBMC" refers to peripheral blood mononuclear cells and includes PBLs (peripheral blood lymphocytes).

Suitably, alterations in expression including changes in gene expression are monitored in samples taken from the mammal or human. Suitable samples include, but are not limited to, tissue samples such as biopsy, blood, urine, buccal scrapes etc. In one case, gene expression is preferably detected in tumour cells, particularly cells derived from a tumour such as breast, lung, gastric, head and neck, colorectal, renal, pancreatic, uterine, hepatic, bladder, endometrial and prostate cancers and leukemias or from blood cells such as lymphocytes and, preferably, peripheral lymphocytes such as PBMC.

In another case altered protein expression is detected in serum or plasma or tissue culture supernatant samples from a mammal or human.

In detection of proteins in serum and, in particular, in plasma samples of patients, samples are removed and subjected to protein analytical techniques such as flow cytometry, ELISA, PET and SELDI-TOF MS, as described herein.

In one preferred case, the method comprises extracting RNA from said sample and detecting gene expression by QPCR.

In another case, gene expression is detected by detecting protein products such as, for example, by Western Blot.

The disclosure also provides a method for identifying an agent capable of inhibiting or reversing epithelial-to-mesenchymal transition (EMT), said method comprising the steps of:
(i) contacting the agent with Axl receptor or cells expressing the Axl receptor;
(ii) measuring the Axl receptor activity in the presence of the agent; and
(iii) comparing the activity measured in step (ii) to that measured under controlled conditions, wherein a decrease identifies the agent as being capable of inhibiting or reversing epithelial-to-mesenchymal transition (EMT).

Preferably, the activity measured is tyrosine phosphorylation of a substrate of the Axl receptor.

More preferably, the activity measured is autophosphorylation of the Axl receptor.

Preferably the cells in the contacting step (i) have previously been transfected by the Axl gene.

Even more preferably, the transfected cells are either transiently or stably transfected.

In one preferred case the controlled conditions in step (iii) comprise contacting the agent with cells which lack an active Axl gene. Even more preferably, the cells have a mutated inactive form of the Axl gene.

In another preferred case the controlled conditions in step (iii) comprise comparing the activity to that measured in the absence of the agent.

In one particularly preferred case the Axl receptor comprises a biologically active portion of the intracellular domain,
In one preferred case the Axl receptor is immobilized, for example, by attachment to a solid phase.

Also disclosed is a method for identifying an agent capable of inhibiting or reversing epithelial-to-mesenchymal transition (EMT) by screening a plurality of agents, said method comprising the steps of:
(i) contacting the plurality of agents with the Axl receptor or cells expressing the Axl receptor;
(ii) measuring the Axl receptor activity in the presence of the plurality of agents;
(iii) comparing the activity measured in step (ii) to that under controlled conditions, wherein a decrease identifies the plurality of agents as being capable of inhibiting or reversing epithelial-to-mesenchymal transition (EMT); and
(v) separately determining which agent or agents present in the plurality inhibit or reverse epithelial-to-mesenchymal transition (EMT).

Preferably, in the methods described above, the agent is for treating metastatic cancer.

### Agents identified by the method

Also disclosed is the use of an agent identified according to any of the above-described methods in the preparation of a medicament for the treatment of metastatic cancer.

Preferably, the cancer is selected from breast, lung, gastric, head and neck, colorectal, renal, pancreatic, uterine, hepatic, bladder, endometrial and prostate cancers and leukemias. More preferably, the cancer is breast cancer.

### Measuring altered expression of gene/protein markers

Levels of gene and protein expression may be determined using a number of different techniques.

### (a) at the RNA level

Gene expression can be detected at the RNA level. RNA may be extracted from cells using RNA extraction techniques including, for example, using acid phenol/guanidine isothiocyanate extraction (RNAzol B; Biogenesis), RNeasy RNA preparation kits (Qiagen) or PAXgene (PreAnalytix, Switzerland). Typical assay formats utilising ribonucleic acid hybridisation include nuclear run-on assays, RT-PCR, RNase protection assays (Melton et al., Nuc. Acids Res. 12:7035), Northern blotting and *In Situ* hybridization. Gene expression can also be detected by microarray analysis as described below.

For Northern blotting, RNA samples are first separated by size via electrophoresis in an agarose gel under denaturing conditions. The RNA is then transferred to a membrane, crosslinked and hybridized with a labeled probe. Nonisotopic or high specific activity radiolabeled probes can be used including random-primed, nick-translated, or PCR-generated DNA probes, *in vitro* transcribed RNA probes, and oligonucleotides. Additionally, sequences with only partial homology (e.g., cDNA from a different species or genomic DNA fragments that might contain an exon) may be used as probes.

Nuclease Protection Assays (including both ribonuclease protection assays and S1 nuclease assays) provide an extremely sensitive method for the detection and quantitation of specific mRNAs. The basis of the NPA is solution hybridization of an antisense probe (radiolabeled or nonisotopic) to an RNA sample. After hybridization, single-stranded, unhybridized probe and RNA are degraded by nucleases. The remaining protected fragments are separated on an acrylamide gel. NPAs allow the simultaneous detection of several RNA species.

*In situ* hybridization (ISH) is a powerful and versatile tool for the localization of specific mRNAs in cells or tissues. Hybridization of the probe takes place within the cell or tissue. Since cellular structure is maintained throughout the procedure, ISH provides information about the location of mRNA within the tissue sample.

The procedure begins by fixing samples in neutral-buffered formalin, and embedding the tissue in paraffin. The samples are then sliced into thin sections and mounted onto microscope slides. Alternatively, tissue can be sectioned frozen and post-fixed in paraformaldehyde. After a series of washes to dewax and rehydrate the sections, a Proteinase K digestion is performed to increase probe accessibility, and a labeled probe is then hybridized to the sample sections. Radiolabeled probes are visualized with liquid film dried onto the slides, while nonisotopically labeled probes are conveniently detected with colorimetric or fluorescent reagents. This latter method of detection is the basis for Fluorescent *In Situ* Hybridisation (FISH).

Methods for detection which can be employed include radioactive labels, enzyme labels, chemiluminescent labels, fluorescent labels and other suitable labels.

Typically, RT-PCR is used to amplify RNA targets. In this process, the reverse transcriptase enzyme is used to convert RNA to complementary DNA (cDNA) which can then be amplified to facilitate detection. Relative quantitative RT-PCR involves amplifying an internal control simultaneously with the gene of interest. The internal control is used to normalize the samples. Once normalized, direct comparisons of relative abundance of a specific mRNA can be made across the samples. Commonly used internal controls include, for example, GAPDH, HPRT, actin and cyclophilin.

Many DNA amplification methods are known, most of which rely on an enzymatic chain reaction (such as a polymerase chain reaction, a ligase chain reaction, or a self-sustained sequence replication) or from the replication of all or part of the vector into which it has been cloned.

Many target and signal amplification (TAS) methods have been described in the literature, for example, general reviews of these methods in Landegren, U. et al., Science 242:229-237 (1988) and Lewis, R., Genetic Engineering News 10:1, 54-55 (1990).

PCR is a nucleic acid amplification method described inter alia in US 4,683,195 and 4,683,202. PCR can be used to amplify any known nucleic acid in a diagnostic context (Mok et al., 1994, Gynaecologic Oncology 52:247-252). Self-sustained sequence replication (3SR) is a variation of TAS, which involves the isothermal amplification of a nucleic acid template via sequential rounds of reverse transcriptase (RT), polymerase and nuclease activities that are mediated by an enzyme cocktail and appropriate oligonucleotide primers (Guatelli et al., 1990, Proc, Natl. Acad. Sci. USA 87:1874). Ligation amplification reaction or ligation amplification system uses DNA ligase and four oligonucleotides, two per target strand. This technique is described by Wu, D. Y. and Wallace, R. B., 1989, Genomics 4:560. In the Qβ Replicase technique, RNA replicase for the bacteriophage Qβ, which replicates single-stranded RNA, is used to amplify the target DNA, as described by Lizardi et al., 1988, Bio/Technology 6:1197.

Quantitative PCR (Q-PCR) is a technique which allows relative amounts of transcripts within a sample to be determined. A suitable method for performing QPCR is described herein.

Alternative amplification technology can be exploited. For example, rolling circle amplification (Lizardi et al., 1998, Nat Genet 19:225) is an amplification technology available commercially (RCAT™) which is driven by DNA polymerase and can replicate circular oligonucleotide probes with either linear or geometric kinetics under isothermal conditions. A further technique, strand displacement amplification (SDA; Walker et al., 1992, Proc. Natl. Acad. Sci. USA 80:392) begins with a specifically defined sequence unique to a specific target.

Suitable probes for detecting the expression of Axl identified herein may conveniently be packaged in the form of a test kit in a suitable container. In such kits the probe may be bound to a solid support where the assay format for which the kit is designed requires such binding. The kit may also contain suitable reagents for treating the sample to be probed, hybridising the probe to nucleic acid in the sample, control reagents, instructions, and the like. Suitable kits may comprise, for example, primers for a QPCR reaction or labelled probes for performing FISH.

### (b) at the polypeptide level

Altered gene or protein expression may also be detected by measuring the polypeptides encoded by the Axl gene. This may be achieved by using molecules which bind to the polypeptides encoded by Axl gene. Suitable molecules/agents which bind either directly or indirectly to the polypeptides in order to detect the presence of the protein include naturally occurring molecules such as peptides and proteins, for example antibodies, or they may be synthetic molecules.

Antibodies for the Axl genes or proteins may be derived from commercial sources or through techniques which are familiar to those skilled in the art. In one case and where altered expression manifests itself through the expression of alteration of post translationally-modified forms of a protein biomarker, antibodies specific for those different forms may be used.

Methods for production of antibodies are known by those skilled in the art. If polyclonal antibodies are desired, a selected mammal (*e.g.,* mouse, rabbit, goat, horse, etc.) is immunised with an immunogenic polypeptide bearing an epitope(s) from a polypeptide. Serum from the immunised animal is collected and treated according to known procedures. If serum containing polyclonal antibodies to an epitope from a polypeptide contains antibodies to other antigens, the polyclonal antibodies can be purified by immunoaffinity chromatography. Techniques for producing and processing polyclonal antisera are known in the art. In order to generate a larger immunogenic response, polypeptides or fragments thereof may be haptenised to another polypeptide for use as immunogens in animals or humans.

Monoclonal antibodies directed against epitopes in polypeptides can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against epitopes in the polypeptides of the disclosure can be screened for various properties; *i.e.,* for isotype and epitope affinity.

An alternative technique involves screening phage display libraries where, for example the phage express scFv fragments on the surface of their coat with a large variety of complementarity determining regions (CDRs). This technique is well known in the art.

As used herein the term "antibody", unless specified to the contrary, includes whole antibodies, or fragments of whole antibodies which retain their binding activity for a target antigen. Such fragments include Fv, F(ab') and F(ab')₂ fragments, as well as single chain antibodies (scFv). Furthermore, the antibodies and fragments thereof may be humanised antibodies, for example as described in EP239400A. The term "antibody" as used herein also encompasses antibody-like affinity reagents. For example: monoclonal and polyclonal antibodies, recombinant antibodies, proteolytic and recombinant fragments of antibodies (Fab, Fv, scFv, diabodies), single-domain antibodies (VHH, sdAb, nanobodies, IgNAR, VNAR), and proteins unrelated to antibodies, which have been engineered to have antibody-like specific binding, such as the following:

| **Name** | **Based on:** | |
|---|---|---|
| Affibodies | Protein A, Z domain | 6 kDa |
| Affitins | Sac7d (from Sulfolobus acidocaldarius) | 7 kDa |
| Anticalins | Lipocalins | 20 kDa |
| DARPins | Ankyrin repeat motif | 14 kDa |
| Fynomers | Fyn, SH3 domain | 7 kDa |
| Kunitz domain peptides | Various protease inhibitors | 6 kDa |
| Monobodies | Fibronectin | |

Standard laboratory techniques such as immunoblotting as described above can be used to detect altered levels of Axl activity, as compared with untreated cells in the same cell population.

Gene expression may also be determined by detecting changes in post-translational processing of polypeptides or post-transcriptional modification of nucleic acids. For example, differential phosphorylation of polypeptides, the cleavage of polypeptides or alternative splicing of RNA, and the like may be measured. Levels of expression of gene products such as polypeptides, as well as their post-translational modification, may be detected using proprietary protein assays or techniques such as 2D polyacrylamide gel electrophoresis.

Antibodies may be used for detecting Axl expression by a method which comprises: (a) providing an antibody (b) incubating a biological sample with said antibody under conditions which allow for the formation of an antibody-antigen complex; and (c) determining whether antibody-antigen complex comprising said antibody is formed.

Suitable samples include extracts of tissues such as brain, breast, ovary, lung, colon, pancreas, testes, liver, muscle and bone tissues or from neoplastic growths derived from such tissues. Other suitable examples include blood or urine samples.

Antibodies that specifically bind to Axl proteins can be used in diagnostic or prognostic methods and kits that are well known to those of ordinary skill in the art to detect or quantify the expression of Axl protein in a body fluid or tissue. Results from these tests can be used to diagnose or predict the occurrence or recurrence of cancer and other cell motility or cell survival-mediated diseases, or to assess the effectiveness of drug dosage and treatment.

Antibodies can be assayed for immunospecific binding by any method known in the art. The immunoassays which can be used include but are not limited to competitive and non-competitive assay systems using techniques such as western blots, immunohistochemistry, radioimmunoassays, ELISA, sandwich immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

Such assays are routine in the art (see, for example, Ausubel et al., eds, 1994, Current Protocols in Molecular Biology, Vol. 1, John Wiley & Sons, Inc., New York,).

Antibodies for use in the disclosure are preferably bound to a solid support and/or packaged into kits in a suitable container along with suitable reagents, controls, instructions and the like.

Other methods include, but are not limited to, 2D-PAGE although this is less suitable for large-scale screening. Newer techniques include matrix-assisted laser desorption ionization time of flight mass spectrometry (MALDI-TOF MS). In MALDI-TOF analysis, proteins in a complex mixture are affixed to a solid metallic matrix, desorbed with a pulsed laser beam to generate gas-phase ions that traverse a field-free flight tube, and are then separated according to their mass-dependent velocities. Individual proteins and peptides can be identified through the use of informatics tools to search protein and peptide sequence databases. Surface-enhanced laser desorption/ionisation time of flight MS (SELDI-TOF MS) is an affinity-based MS method in which proteins are selectively adsorbed to a chemically modified solid surface, impurities are removed by washing, an energy-absorbing matrix is applied, and the proteins are identified by laser desorption mass analysis.

SELDI-TOF-MS can be used for the detection of the appearance/loss of either intact proteins or fragments of specific proteins. In addition SELDI-TOF-MS can also be used for detection of post translational modifications of proteins due to the difference in mass caused by the addition/removal of chemical groups. Thus phosphorylation of a single residue will cause a mass shift of 80 Da due to the phosphate group. A data base of molecular weights that can be attributed to post-translational modifications is freely accessible on the internet (http://www.abrf.org/index.cfm/dm.home?avgmass=all). Moreover specific polypeptides can be captured by affinity-based approaches using SELDI-TOF-MS by employing antibodies that specifically recognise a post-translationally modified form of the protein, or that can recognise all forms of the protein equally well.

### Arrays

Array technology and the various techniques and applications associated with it is described generally in numerous textbooks and documents. These include Lemieux et al., 1998, Molecular Breeding 4:277-289; Schena and Davis. Parallel Analysis with Biological Chips. in PCR Methods Manual (eds. M. Innis, D. Gelfand, J. Sninsky); Schena and Davis, 1999, Genes, Genomes and Chips. In DNA Microarrays: A Practical Approach (ed. M. Schena), Oxford University Press, Oxford, UK, 1999); The Chipping Forecast (Nature Genetics special issue; January 1999 Supplement); Mark Schena (Ed.), Microarray Biochip Technology, (Eaton Publishing Company); Cortes, 2000, The Scientist 14(17):25; Gwynne and Page, Microarray analysis: the next revolution in molecular biology, Science, 1999, August 6; Eakins and Chu, 1999, Trends in Biotechnology, 17:217-218, and also at various world wide web sites.

Array technology overcomes the disadvantages with traditional methods in molecular biology, which generally work on a "one gene in one experiment" basis, resulting in low throughput and the inability to appreciate the "whole picture" of gene function. Currently, the major applications for array technology include the identification of sequence (gene / gene mutation) and the determination of expression level (abundance) of genes. Gene expression profiling may make use of array technology, optionally in combination with proteomics techniques (Celis et al., 2000, FEBS Lett, 480(1):2-16; Lockhart and Winzeler, 2000, Nature 405(6788):827-836; Khan *et al.,* 1999, 20(2):223-9). Other applications of array technology are also known in the art; for example, gene discovery, cancer research (Marx, 2000, Science 289: 1670-1672; Scherf et alet al., 2000, Nat Genet 24(3):236-44; Ross et al., 2000, Nat Genet 2000, 24(3):227-35), SNP analysis (Wang et al., 1998, Science 280(5366): 1077-82), drug discovery, pharmacogenomics, disease diagnosis (for example, utilising microfluidics devices: Chemical & Engineering News, February 22, 1999, 77(8):27-36), toxicology (Rockett and Dix (2000), Xenobiotica 30(2):155-77; Afshari et al., 1999, Cancer Res 59(19):4759-60) and toxicogenomics (a hybrid of functional genomics and molecular toxicology). The goal of toxicogenomics is to find correlations between toxic responses to toxicants and changes in the genetic profiles of the objects exposed to such toxicants (Nuwaysir et al., 1999, Molecular Carcinogenesis 24:153-159).

In the context of the present disclosure array technology can be used, for example, in the analysis of the expression of Axl protein. In one case array technology may be used to assay the effect of a candidate compound on Axl activity.

In general, any library or group of samples may be arranged in an orderly manner into an array, by spatially separating the members of the library or group. Examples of suitable libraries for arraying include nucleic acid libraries (including DNA, cDNA, oligonucleotide, etc. libraries), peptide, polypeptide and protein libraries, as well as libraries comprising any molecules, such as ligand libraries, among others. Accordingly, where reference is made to a "library" in this document, unless the context dictates otherwise, such reference should be taken to include reference to a library in the form of an array.

The samples (e.g., members of a library) are generally fixed or immobilised onto a solid phase, preferably a solid substrate, to limit diffusion and admixing of the samples. In a preferred case libraries of DNA binding ligands may be prepared. In particular, the libraries may be immobilised to a substantially planar solid phase, including membranes and non-porous substrates such as plastic and glass. Furthermore, the samples are preferably arranged in such a way that indexing (*i.e*., reference or access to a particular sample) is facilitated. Typically the samples are applied as spots in a grid formation. Common assay systems may be adapted for this purpose. For example, an array may be immobilised on the surface of a microplate, either with multiple samples in a well, or with a single sample in each well. Furthermore, the solid substrate may be a membrane, such as a nitrocellulose or nylon membrane (for example, membranes used in blotting experiments). Alternative substrates include glass, or silica based substrates. Thus, the samples are immobilised by any suitable method known in the art, for example, by charge interactions, or by chemical coupling to the walls or bottom of the wells, or the surface of the membrane. Other means of arranging and fixing may be used, for example, pipetting, drop-touch, piezoelectric means, ink-jet and bubblejet technology, electrostatic application, etc. In the case of silicon-based chips, photolithography may be utilised to arrange and fix the samples on the chip.

The samples may be arranged by being "spotted" onto the solid substrate; this may be done by hand or by making use of robotics to deposit the sample. In general, arrays may be described as macroarrays or microarrays, the difference being the size of the sample spots. Macroarrays typically contain sample spot sizes of about 300 microns or larger and may be easily imaged by existing gel and blot scanners. The sample spot sizes in microarrays are typically less than 200 microns in diameter and these arrays usually contain thousands of spots. Thus, microarrays may require specialized robotics and imaging equipment, which may need to be custom made. Instrumentation is described generally in a review by Cortese, 2000, The Scientist 14(11):26.

Techniques for producing immobilised libraries of DNA molecules have been described in the art. Generally, most prior art methods described how to synthesise single-stranded nucleic acid molecule libraries, using for example masking techniques to build up various permutations of sequences at the various discrete positions on the solid substrate. US 5,837,832, describes an improved method for producing DNA arrays immobilised to silicon substrates based on very large scale integration technology. In particular, US 5,837,832 describes a strategy called "tiling" to synthesize specific sets of probes at spatially-defined locations on a substrate which may be used to produced the immobilised DNA libraries. US 5,837,832 also provides references for earlier techniques that may also be used.

Arrays of peptides (or peptidomimetics) may also be synthesised on a surface in a manner that places each distinct library member (*e.g.,* unique peptide sequence) at a discrete, predefined location in the array. The identity of each library member is determined by its spatial location in the array. The locations in the array where binding interactions between a predetermined molecule (*e.g.,* a target or probe) and reactive library members occur is determined, thereby identifying the sequences of the reactive library members on the basis of spatial location. These methods are described in US 5,143,854; WO 90/15070 and WO 92/10092; Fodor et al., 1991, Science 251:767; Dower and Fodor, 1991, Ann. Rep. Med. Chem. 26:271.

To aid detection, targets and probes may be labelled with any readily detectable reporter, for example, a fluorescent, bioluminescent, phosphorescent, radioactive, etc reporter. Such reporters, their detection, coupling to targets/probes, etc are discussed elsewhere in this document. Labelling of probes and targets is also disclosed in Shalon et al., 1996, Genome Res 6(7):639-45.

Specific examples of DNA arrays include the following:
Format I: probe cDNA (∼500 - ∼5,000 bases long) is immobilized to a solid surface such as glass using robot spotting and exposed to a set of targets either separately or in a mixture. This method is widely considered as having been developed at Stanford University (Ekins and Chu, 1999, Trends in Biotechnology, 17:217-218).
Format II: an array of oligonucleotide (∼20 - ∼25-mer oligos) or peptide nucleic acid (PNA) probes is synthesized either *in situ* (on-chip) or by conventional synthesis followed by on-chip immobilization. The array is exposed to labeled sample DNA, hybridized, and the identity/abundance of complementary sequences are determined. Such a DNA chip is sold by Affymetrix, Inc., under the GeneChip® trademark.

Examples of some commercially available microarray formats are set out, for example, in Marshall and Hodgson, 1998, Nature Biotechnology 16(1):27-31.

Data analysis is also an important part of an experiment involving arrays. The raw data from a microarray experiment typically are images, which need to be transformed into gene expression matrices - tables where rows represent for example genes, columns represent for example various samples such as tissues or experimental conditions, and numbers in each cell for example characterize the expression level of the particular gene in the particular sample. These matrices have to be analyzed further, if any knowledge about the underlying biological processes is to be extracted. Methods of data analysis (including supervised and unsupervised data analysis as well as bioinformatics approaches) are disclosed in Brazma and Vilo J, 2000, FEBS Lett 480(1):17-24.

As disclosed above, proteins, polypeptides, etc may also be immobilised in arrays. For example, antibodies have been used in microarray analysis of the proteome using protein chips (Borrebaeck CA, 2000, Immunol Today 21(8):379-82). Polypeptide arrays are reviewed in, for example, MacBeath and Schreiber, 2000, Science, 289(5485):1760-1763.

### Pharmaceutical Composition

Disclosed herein is a pharmaceutical composition comprising an agent identified according to any of the above-described methods admixed with a pharmaceutically acceptable diluent, excipient or carrier.

Also disclosed is a process of preparing a composition which comprises:
(i) identifying an agent capable of inhibiting or reversing epithelial-to-mesenchymal transition (EMT) using any of the above-described methods; and
(ii) admixing said agent with a pharmaceutically acceptable diluent, carrier or excipient.

Also disclosed herein is the use of an agent identified by the methods of the disclosure in the preparation of a medicament for treating a proliferative disorder, more preferably, cancer.

the agent identified by the above methods may be presented as a pharmaceutical formulation, comprising the compounds or physiologically acceptable salt, ester or other physiologically functional derivative thereof, together with one or more pharmaceutically acceptable carriers and optionally other therapeutic and/or prophylactic ingredients. The carrier(s) must be acceptable in the sense of being compatible with the other ingredients of the formulation and not deleterious to the recipient thereof. The pharmaceutical compositions may be for human or animal usage in human and veterinary medicine.

Examples of such suitable excipients for the various different forms of pharmaceutical compositions described herein may be found in the "Handbook of Pharmaceutical Excipients", 2nd Edition, (1994), Edited by A Wade and PJ Weller.

Acceptable carriers or diluents for therapeutic use are well known in the pharmaceutical art, and are described, for example, in Remington's Pharmaceutical Sciences, Mack Publishing Co. (A. R. Gennaro edit. 1985).

Examples of suitable carriers include lactose, starch, glucose, methyl cellulose, magnesium stearate, mannitol, sorbitol and the like. Examples of suitable diluents include ethanol, glycerol and water.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s), buffer(s), flavouring agent(s), surface active agent(s), thickener(s), preservative(s) (including anti-oxidants) and the like, and substances included for the purpose of rendering the formulation isotonic with the blood of the intended recipient.

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride and the like.

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.
Pharmaceutical formulations include those suitable for oral, topical (including dermal, buccal and sublingual), rectal or parenteral (including subcutaneous, intradermal, intramuscular and intravenous), nasal and pulmonary administration e.g., by inhalation. The formulation may, where appropriate, be conveniently presented in discrete dosage units and may be prepared by any of the methods well known in the art of pharmacy. All methods include the step of bringing into association an active compound with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of active agent. A tablet may be made by compression or moulding, optionally with one or more accessory ingredients. Compressed tablets may be prepared by compressing in a suitable machine an active agent in a free-flowing form such as a powder or granules optionally mixed with a binder, lubricant, inert diluent, lubricating agent, surface-active agent or dispersing agent. Moulded tablets may be made by moulding an active agent with an inert liquid diluent. Tablets may be optionally coated and, if uncoated, may optionally be scored. Capsules may be prepared by filling an active agent, either alone or in admixture with one or more accessory ingredients, into the capsule shells and then sealing them in the usual manner. Cachets are analogous to capsules wherein an active agent together with any accessory ingredient(s) is sealed in a rice paper envelope. An active agent may also be formulated as dispersible granules, which may for example be suspended in water before administration, or sprinkled on food. The granules may be packaged, e.g., in a sachet. Formulations suitable for oral administration wherein the carrier is a liquid may be presented as a solution or a suspension in an aqueous or non-aqueous liquid, or as an oil-in-water liquid emulsion.

Formulations for oral administration include controlled release dosage forms, e.g., tablets wherein an active agent is formulated in an appropriate release - controlling matrix, or is coated with a suitable release - controlling film. Such formulations may be particularly convenient for prophylactic use.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of an active agent with the softened or melted carrier(s) followed by chilling and shaping in moulds.

Pharmaceutical formulations suitable for parenteral administration include sterile solutions or suspensions of an active agent in aqueous or oleaginous vehicles.

Injectable preparations may be adapted for bolus injection or continuous infusion. Such preparations are conveniently presented in unit dose or multi-dose containers which are sealed after introduction of the formulation until required for use. Alternatively, an active agent may be in powder form which is constituted with a suitable vehicle, such as sterile, pyrogen-free water, before use.

An active compound may also be formulated as long-acting depot preparations, which may be administered by intramuscular injection or by implantation, e.g., subcutaneously or intramuscularly. Depot preparations may include, for example, suitable polymeric or hydrophobic materials, or ion-exchange resins. Such long-acting formulations are particularly convenient for prophylactic use.

Formulations suitable for pulmonary administration via the buccal cavity are presented such that particles containing an active compound and desirably having a diameter in the range of 0.5 to 7 microns are delivered in the bronchial tree of the recipient.

As one possibility such formulations are in the form of finely comminuted powders which may conveniently be presented either in a pierceable capsule, suitably of, for example, gelatin, for use in an inhalation device, or alternatively as a self-propelling formulation comprising an active agent, a suitable liquid or gaseous propellant and optionally other ingredients such as a surfactant and/or a solid diluent. Suitable liquid propellants include propane and the chlorofluorocarbons, and suitable gaseous propellants include carbon dioxide. Self-propelling formulations may also be employed wherein an active agent is dispensed in the form of droplets of solution or suspension.

Such self-propelling formulations are analogous to those known in the art and may be prepared by established procedures. Suitably they are presented in a container provided with either a manually-operable or automatically functioning valve having the desired spray characteristics; advantageously the valve is of a metered type delivering a fixed volume, for example, 25 to 100 microlitres, upon each operation thereof.

As a further possibility an active agent may be in the form of a solution or suspension for use in an atomizer or nebuliser whereby an accelerated airstream or ultrasonic agitation is employed to produce a fine droplet mist for inhalation.

Formulations suitable for nasal administration include preparations generally similar to those described above for pulmonary administration. When dispensed such formulations should desirably have a particle diameter in the range 10 to 200 microns to enable retention in the nasal cavity; this may be achieved by, as appropriate, use of a powder of a suitable particle size or choice of an appropriate valve. Other suitable formulations include coarse powders having a particle diameter in the range 20 to 500 microns, for administration by rapid inhalation through the nasal passage from a container held close up to the nose, and nasal drops comprising 0.2 to 5% w/v of an active agent in aqueous or oily solution or suspension.

Pharmaceutically acceptable carriers are well known to those skilled in the art and include, but are not limited to, 0.1 M and preferably 0.05 M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

Formulations suitable for topical formulation may be provided for example as gels, creams or ointments. Such preparations may be applied e.g. to a wound or ulcer either directly spread upon the surface of the wound or ulcer or carried on a suitable support such as a bandage, gauze, mesh or the like which may be applied to and over the area to be treated.

Liquid or powder formulations may also be provided which can be sprayed or sprinkled directly onto the site to be treated, e.g. a wound or ulcer. Alternatively, a carrier such as a bandage, gauze, mesh or the like can be sprayed or sprinkle with the formulation and then applied to the site to be treated.

The disclosure also provides a process for the preparation of a pharmaceutical or veterinary composition as described above, the process comprising bringing the active compound(s) into association with the carrier, for example by admixture.

In general, the formulations are prepared by uniformly and intimately bringing into association the active agent with liquid carriers or finely divided solid carriers or both, and then if necessary shaping the product. The disclosure extends to methods for preparing a pharmaceutical composition comprising bringing an agent into association with a pharmaceutically or veterinarily acceptable carrier or vehicle.

### Therapeutic Applications

Also disclosed herein is a method of inhibiting growth and spread of cancer cells and other cells having undergone an epithelial-to-mesenchymal transition (EMT) in a subject in need thereof, said method comprising administering an Axl inhibitor to said subject.

As used herein "inhibiting epithelial-to-mesenchymal transition" refers to a decrease in the number of cells undergoing and having undergone epithelial-to-mesenchymal transition (EMT) in a subject.

In one preferred case the subject in need thereof is suffering from cancer.

Preferably, where the subject is suffering from cancer, the method inhibits tumor cells having undergone an epithelial-to-mesenchymal transition (EMT) to such an extent that the cancer cells are unable to metastasise, i.e. the inhibition is such that the cancer does not develop into metastatic cancer, or that cells already metastasized are unable to grow at distant sites in the body.

In one highly preferred case the method prevents cells that have completed epithelial-to-mesenchymal transition (EMT) from spreading to distant sites in the body, i.e. the inhibition is such that the spread of metastatic cells dependent on epithelial-to-mesenchymal transition (EMT) is eliminated.

Also disclosed is a method for treating metastatic cancer in a subject in need thereof, said method comprising inhibiting tumor cells undergoing epithelial-to-mesenchymal transition (EMT) by administering to said subject an Axl inhibitor.

As used herein, the term "Axl inhibitor" refers to a molecule that is capable of inhibiting Axl, the Axl signalling pathway or any one or more components of the Axl signalling pathway. In one case the molecule will be capable of reducing or preventing Axl or Axl protein expression.

In one particularly preferred case the Axl inhibitor is an anti-Axl antibody.

In one particularly preferred case the Axl inhibitor is a small molecule kinase inhibitor. An example of such a small molecule inhibitor is R428, as described in Holland *et al* 2010.

In one particularly preferred case the subject is a mammal, more preferably, a human.

Preferably, the cancer is breast, prostate, glioma, lung, pancreatic cancer.

Metastasis accounts for 90% of cancer related mortality. Understanding of the molecular mechanisms that enable tumor cell metastasis is a major health issue. The present inventors have demonstrated that the receptor tyrosine kinase Axl is a strong predictor of poor overall survival of patients following primary mammography-detected breast cancer.

Metastatic breast cancer cells require Axl expression to maintain an invasive malignant phenotype and to form breast tumors in different micrenvironments. Induction of an epithelial-to-mesenchymal transition (EMT) in mammary epithelial cells upregulates Axl expression generating an autocrine signaling loop with its ligand, Gas6. Inhibition of Axl expression prevents metastatic spread from orthotopic sites in the mammary gland to lymph nodes and major organs. Hence inappropriate EMT-dependent activation of Axl during early malignant transitions may promote metastasis and negatively affect overall patient survival. Disruption of Axl signaling via established therapeutic strategies therefore represents an exciting avenue for the therapeutic development of treatments for cell proliferative disorders such as breast cancer, and cell-motility and cell survival-mediated disorders.

Disclosed herein is the use of an Axl inhibitor in the preparation of a medicament for inhibiting or reversing epithelial-to-mesenchymal transition (EMT).

Also disclosed is the use of an Axl inhibitor in the preparation of a medicament for treating metastatic cancer by inhibiting tumor cells dependent on an epithelial-to-mesenchymal transition (EMT).

Also disclosed is a method of treating metastatic cancer or late stage cancer in a subject in need thereof, said method comprising administering an Axl inhibitor to said subject.

Also disclosed is the use of an Axl inhibitor in the preparation of a medicament for treating metastatic cancer or late stage cancer.

Also disclosed is a method of inhibiting metastasis in a subject in need thereof, said method comprising administering an Axl inhibitor to said subject.

Also disclosed is a method of inhibiting EMT-induced invasiveness in subject suffering from cancer, said method comprising administering an Axl inhibitor to said subject.

Also disclosed is the use of an Axl inhibitor in the preparation of a medicament for inhibiting EMT-induced invasiveness in subject suffering from cancer.

Preferably, the Axl inhibitor is an anti-Axl antibody or small molecule inhibitor.

### Administration

The pharmaceutical compositions disclosed herein may be adapted for rectal, nasal, intrabronchial, topical (including buccal and sublingual), vaginal or parenteral (including subcutaneous, intramuscular, intravenous, intraarterial and intradermal), intraperitoneal or intrathecal administration. Preferably the formulation is an orally administered formulation. The formulations may conveniently be presented in unit dosage form, i.e., in the form of discrete portions containing a unit dose, or a multiple or sub-unit of a unit dose. By way of example, the formulations may be in the form of tablets and sustained release capsules, and may be prepared by any method well known in the art of pharmacy.

Formulations for oral administration may be presented as: discrete units such as capsules, gellules, drops, cachets, pills or tablets each containing a predetermined amount of the active agent; as a powder or granules; as a solution, emulsion or a suspension of the active agent in an aqueous liquid or a non-aqueous liquid; or as an oil-in-water liquid emulsion or a water-in-oil liquid emulsion; or as a bolus etc. Preferably, these compositions contain from 1 to 250 mg and more preferably from 10-100 mg, of active ingredient per dose.

For compositions for oral administration (e.g. tablets and capsules), the term "acceptable carrier" includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring and the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

Other forms of administration comprise solutions or emulsions which may be injected intravenously, intraarterially, intrathecally, subcutaneously, intradermally, intraperitoneally or intramuscularly, and which are prepared from sterile or sterilisable solutions. Injectable forms typically contain between 10 - 1000 mg, preferably between 10 - 250 mg, of active ingredient per dose.

The pharmaceutical compositions may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

### Dosage

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific agent employed, the metabolic stability and length of action of that agent, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited,
an effective amount of agent may be administered to inhibit Axl. Of course, this dosage amount will further be modified according to the type of administration of the agent. For example, to achieve an "effective amount" for acute therapy, parenteral administration is preferred. An intravenous infusion of the compound in 5% dextrose in water or normal saline, or a similar formulation with suitable excipients, is most effective, although an intramuscular bolus injection is also useful. Typically, the parenteral dose will be about 0.01 to about 100 mg/kg; preferably between 0.1 and 20 mg/kg, in a manner to maintain the concentration of drug in the plasma at a concentration effective to inhibit a kinase. The agents may be administered one to four times daily at a level to achieve a total daily dose of about 0.4 to about 400 mg/kg/day. The precise amount of an active agent which is therapeutically effective, and the route by which such agent is best administered, is readily determined by one of ordinary skill in the art by comparing the blood level of the agent to the concentration required to have a therapeutic effect.

The agents disclosed herein may also be administered orally to the patient, in a manner such that the concentration of drug is sufficient to achieve one or more of the therapeutic indications disclosed herein. Typically, a pharmaceutical composition containing the agent is administered at an oral dose of between about 0.1 to about 50 mg/kg in a manner consistent with the condition of the patient. Preferably the oral dose would be about 0.5 to about 20 mg/kg.

The agents disclosed herein may be tested in one of several biological assays to determine the concentration of an agent which is required to have a given pharmacological effect.

### Kit of Parts

Also disclosed herein is a kit for assessing the ability of an agent to inhibit or reverse epithelial-to-mesenchymal transition (EMT), said kit comprising anti-Axl antibodies.

Also disclosed is a kit for assessing the ability of an agent to inhibit or reverse epithelial-to-mesenchymal transition (EMT), said kit comprising a nucleic acid probe for Axl.

Also disclosed is a kit for assessing the ability of an agent to inhibit or reverse epithelial-to-mesenchymal transition (EMT), said kit comprising at least one QPCR primer for Axl.

Also disclosed is the use of a kit as defined above in any of the above-described methods.

### Diagnostics and Prognostics

The present disclosure also relates to the use of Axl as a biomarker in the diagnosis or prognosis of diseases characterized by proliferative activity, particularly in individuals being treated with Axl inhibitors.

As used herein, the term "prognostic method" means a method that enables a prediction regarding the progression of a disease of a human or animal diagnosed with the disease, in particular, cancer. More specifically, the cancers of interest include breast, lung, gastric, head and neck, colorectal, renal, pancreatic, uterine, hepatic, bladder, endometrial and prostate cancers and leukemias.

The term "diagnostic method" as used herein means a method that enables a determination of the presence or type of cancer in or on a human or animal. Suitably the marker allows the success of treatment with an Axl inhibitor to be assessed. As discussed above, suitable diagnostics include probes directed to any of the genes as identified herein such as, for example, QPCR primers, FISH probes and so forth.

The term "prognostic method" as used herein means a method that enables a determination of the likelihood of a subject being susceptible or responsive to treatment with a particular agent/regimen. Such prognostic methods provide information on the likely outcome of a particular treatment regimen, for example, the likelihood of a subject responding to said treatment, and/or information as to how aggressively an individual should be treated within a particular treatment regimen, and/or how aggressively an individual should be treated with conventional therapeutic methods such as radiation/chemotherapy. The prognostic methods described herein therefore have important applications in the field of personalised medicines.

One preferred case thus relates to the use of a biomarker as described above in a personalised medicine application.

In one preferred case the personalised medicine application is for determining whether a subject will be susceptible or responsive to treatment with an Axl inhibitor.

In one preferred case the personalised medicine application is for determining whether a subject is particularly likely to suffer from metastatic cancer.

Disclosed herein is a prognostic method for determining whether a subject will be susceptible to treatment with an Axl inhibitor, said method comprising detecting the occurrence of epithelial-to-mesenchymal transition (EMT) in said subject.

Also disclosed is the use of Axl as a biomarker in a prognostic agent for determining whether a subject will be susceptible or responsive to treatment with an Axl inhibitor.

Also disclosed is a prognostic method for determining whether a subject is particularly likely to suffer from metastatic cancer, said method comprising detecting the occurrence of epithelial-to-mesenchymal transition (EMT) in said subject.

Preferably, the prognostic methods described above comprise the steps of:
(i) obtaining a sample from said subject; and
(ii) determining the expression of Axl in said sample as compared to a control sample, wherein upregulation of Axl expression relative to the control sample is indicative of susceptibility to treatment with an Axl inhibitor and increased likelihood of suffering from metastatic cancer.

Throughout the specification, preferably the methods described herein are performed ex *vivo.*

Preferably, the sample is analysed by protein analysis, more preferably, by ELISA, PET, flow cytometry, SELDI-TOF MS or 2-D PAGE.

The disclosure is further illustrated by way of the following non-limiting examples, and with reference to the following Figures, wherein:

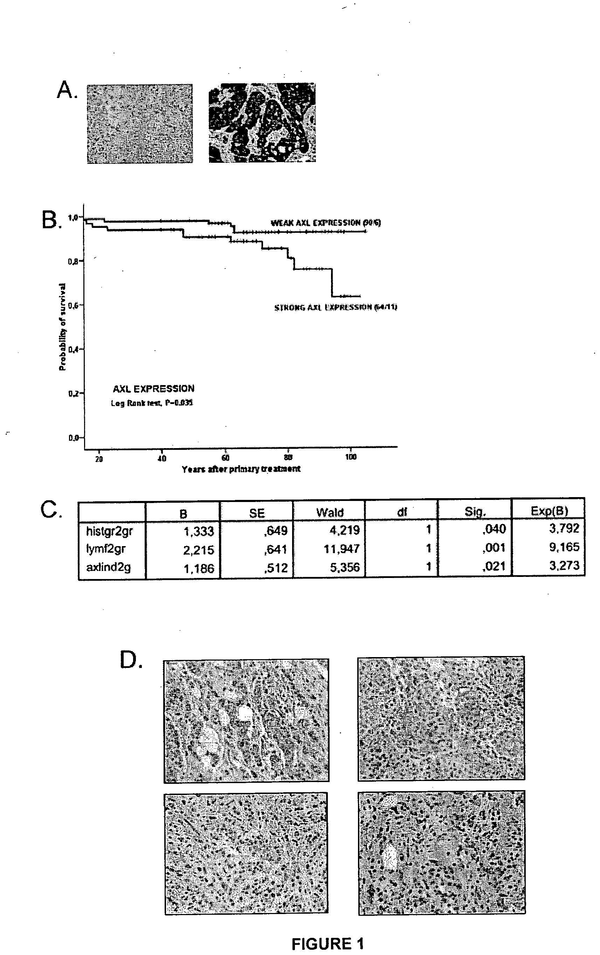

Figure 1 shows that Axl expression is a negative prognostic factor for breast cancer survival. (A) Immunochemistry weak (60 %) and strong Axl expression. (B) Kaplan-Meier analysis of 8 year clinical follow-up. (C) multivariate analysis. (D) Axl expression in matched pairs (n=16) of primary and metastatic human breast carcinoma: primary tumors to the left (*upper.* Axl negative, *lower.* Axl positive), metastases to the right (*upper,* liver, *lower.* bone). Axl expression tended to be stronger in metastases when compared with corresponding primary tumors (p=0.11, McNemar's test).

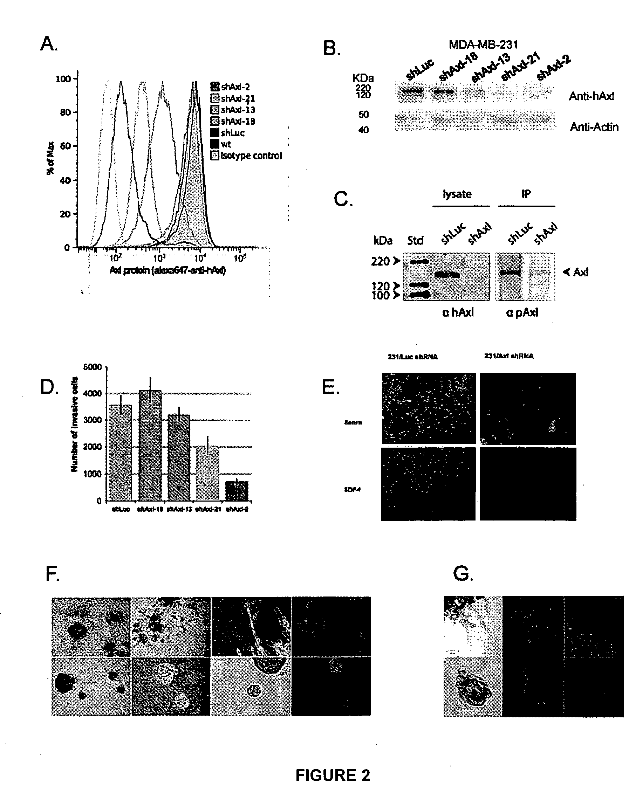

Figure 2 shows that Axl is required for breast cancer cell invasiveness (A) FACS and (B) Western blot analysis of Axl expression in epi-allelic MB-MDA-231 breast carcinoma cell series. (C) Axl is phosphorylated Epi-allelic analysis of matrigel invasion assay induced by serum (D) or SDF-1 (E). 3D matrigel analysis (F, G) of MB-MDA-231/shLuc (upper panels) and MB-MDA-231/shAxl2 (lower panels).

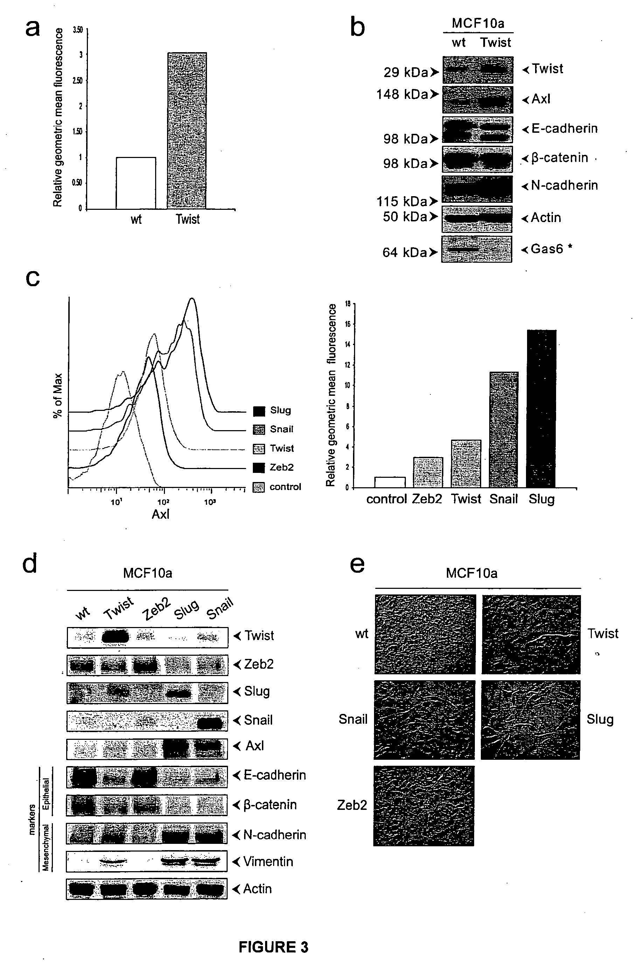

Figure 3 shows Axl activity is upregulated by EMT inducers in breast epithelial cell. (A) Flow cytometry analysis of surface levels of Axl on an MCF10a cell line that stably expresses Twist. (B) Extracts from control (wt) and Twist-expressing MCF10a cells were analysed for changes in epithelial (E-cadherin, β-catenin) and mesenchymal (N-cadherin) markers. *Conditioned medium was analysed by SDS-PAGE and immunoblotting using an antibody against Gas6. (C) MCF10a cells transduced with retroviral vectors encoding Twist, Zeb2, Slug, Snail or vector control (GFP) were analysed on by flow cytometry for Axl surface expression (*left*) and geometric mean fluorescence (right). (D) Extracts from MCF10a cells transduced with Twist, Zeb2, Slug or Snail retroviral vectors were analyzed by immunoblotting for changes in epithelial (E-cadherin, β-catenin) and mesenchymal (Ncadherin, vimentin) markers. (E) Morphology of MCF10a cells transduced with Twist, Zeb2, Slug or Snail retroviral vectors at 72 hours post-seeding.

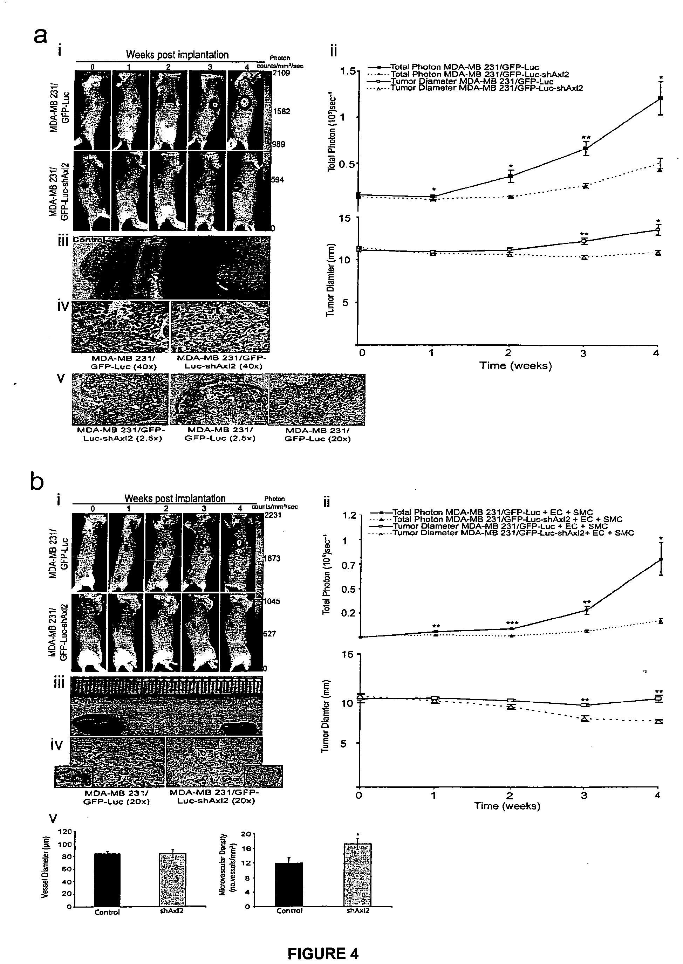

Figure 4 shows that tissue engineered breast tumours require Axl expression. (A) *In vivo* imaging of MDA-MB-231/GFP-Luc tumor tissue engineering implants in NODSCID mice. Temporal tumor growth was monitored by *in vivo* optical imaging of luciferase bioluminescence from MDA-MB-231/GFP-Luc cells (*i*). Tumor cell number (total photon) and extent of radial infiltration (signal diameter) measurements are from control implants (*solid line*) and cell implants expressing MDA-MB-231/GFP-Luc-shAxl2 (*dashed line*) show Axl-dependence of tumor growth and colonization within poly-lactic acid tissue engineering scaffolds (*ii*). Appearance of bilateral scaffolds upon excision (*iii*). Immunohistochemistry analysis of tissue engineered tumors with anti-human Axl at 28 days post-implantation (*left panel,* vector control; *right panel,* shAxl2) (*iv*). Tumor tissue engineering implants with wildtype control (*left panel*) and shAxl2-expressing cells (*right panel*) were analyzed by immunohistochemistry with anti-human Axl (v). Black squares demarcate colonization and radial spread of MDA-MB-231/GFP Luc cells. *p<0.05, **p<0.005 (paired t-test) compared with control. N = 6 mice/group. (B) Temporal *in vivo* imaging of tricellular implants comprising primary human microvascular cells (EC), vascular smooth muscle cells (SMC) and MDA-MB-231/GFP Luc cells (*i*). Tumor growth (total photon) and radial spread (diameter) measurements in control (*solid line*) and shAxl2 expressing MDA-MB-231 cell implants (*broken line*) analyzed in the presence of a tissue engineered vasculature (*ii*). Excised tumors (28 days) are highly vascularized by engineered human microvessels (*iii*). Immunohistochemistry analysis shows that the engineered anti-human CD31-staining vessels contain intra-lumenal red blood cells (*inset*) indicative of patency and perfusion (*iv*: *left panel,* vector control; *right panel,* shAxl- 2). *p<0.05, **p<0.005, ***p<0.0005 (paired t-test) compared with control. *N*= 7 mice/group, Intrascaffold vessel diameter (*left*) is unaffected while microvascular density (*right*) is slightly enhanced in tissue engineered MDA-MB-231 tumors inhibited by shAxl2 expression.

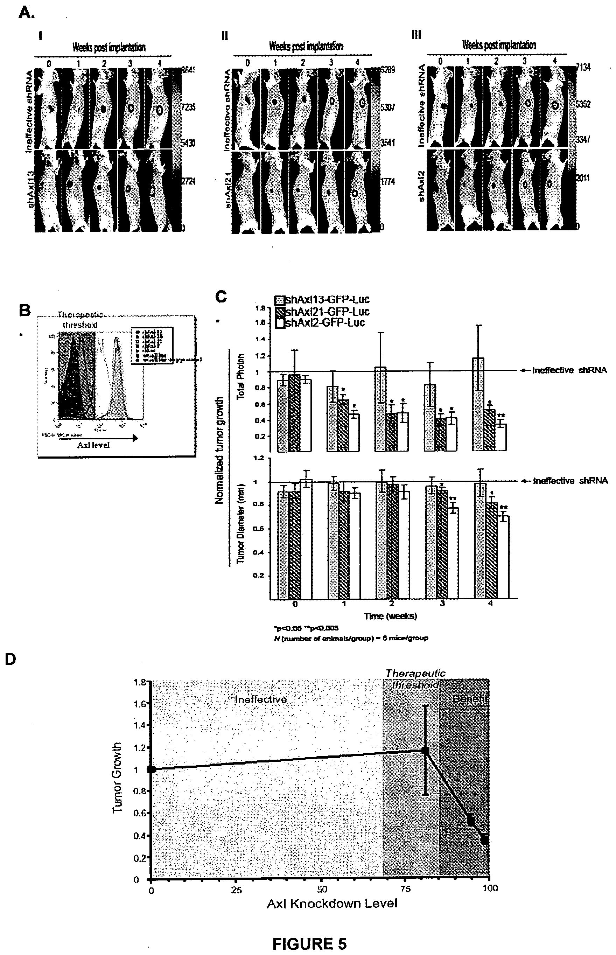

Figure 5 shows in vivo epi-allelic analysis reveals a distinct Axl expression threshold required for breast tumour formation. (A) Temporal *in vivo* imaging of bioluminescence from subcutaneous epi-allelic MDA-MB-231/GFP-Luc xenografts in NOD/SCID !2m*null* mice comprising graded Axl expression by Axl-targeting shRNAs shAxl278 (*i*), shAx280 (*ii*) and shAxl2 (*iii*) compared to an ineffective Axl-targeting shRNA (shAxl279) control. (C) Bar graphs show mean changes in photons and tumour diameters based on optical imaging analysis of tumours. Epi-allelic MDA-MB-231/GFP-Luc tumor growth (*total photon*) and radial infiltration (*signal diameter*) measurements were normalized to shAxl279 (*ineffective shRNA*) (*ii*). (D) Tumor growth (28 day measurements) plotted versus Axl knockdown reveal the therapeutic threshold (80% reduced expression). *p<0.05, **p<0.005 (paired t-test) compared with control. *N* = 6 mice/group.

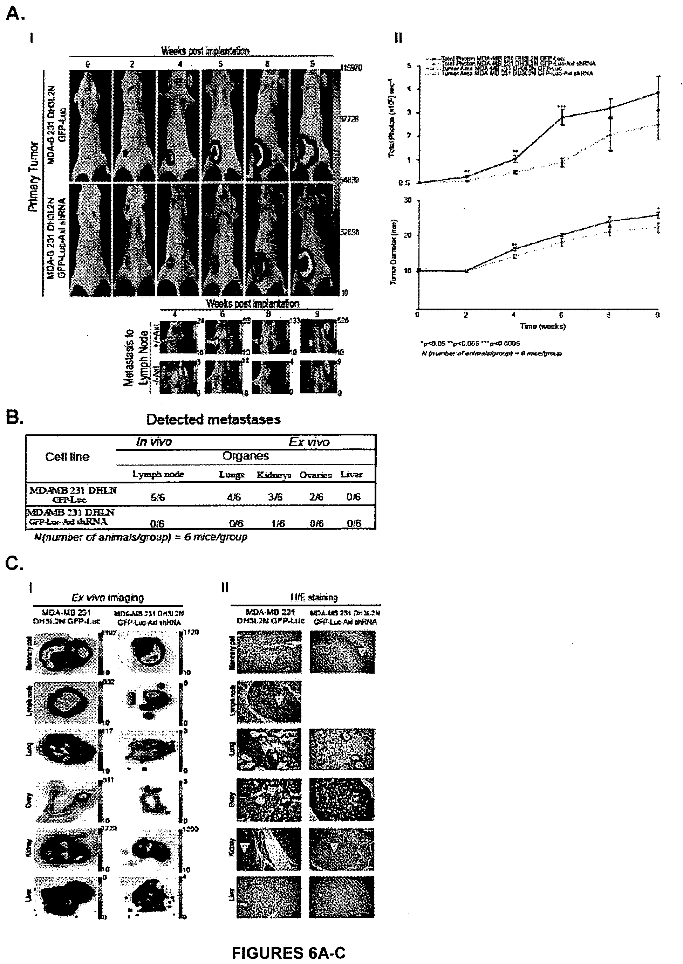

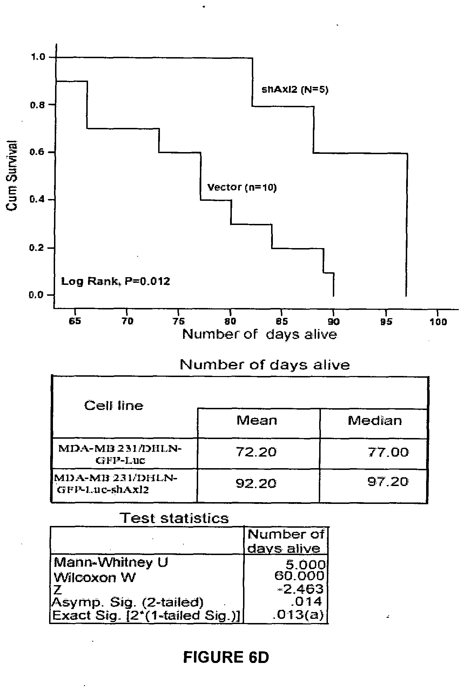

Figure 6 shows that Axl is required for metastasis of breast carcinoma cells. (A) *In vivo* monitored primary tumour and metastasis, I, Orthotropic growth (upper panel) and metastasis to lymph node over time (lower panel). II, Primary tumour growth monitored by *in vivo* optical imaging (GE Explore Optix) of wild type (solid line) and Axl RNAi implants (broken line). (B) Detection of metastasis in different organs. (C) *Ex vivo* detection of breast cancer cells. I, Images of excised organs are shown. II, Histological analysis of the same tissues confirmed metastasis (arrow) to different organs. (D) Mann-Whitney (log-rank) test between control (vector) and shAxl2 orthographically injected mice shows an increasing of survival in MDA-MB-231-D3H2LN/GFP-Luc-shAxl2 tumor-bearing mice (P=0.013):

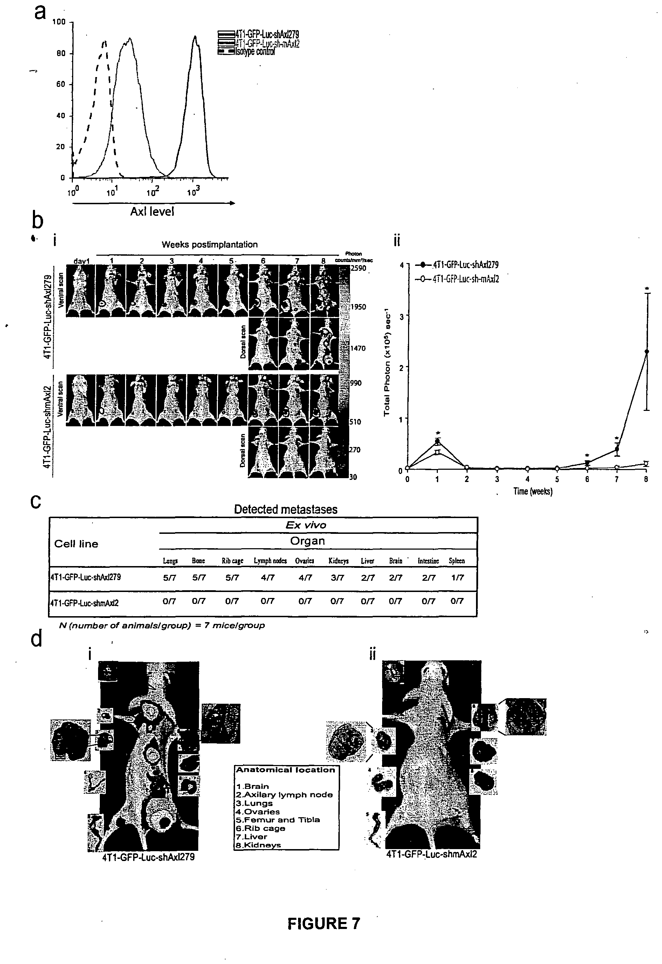

Figure 7 shows that Axl is essential for post-immune response recurrence and metastasis of syngeneic breast carcinoma cells in BALB/c mice. (A) 4T1-GFP-Luc mouse breast carcinoma cells expressing mouse Axl-targeting shRNA (shmAxl2) or human Axl-targeting shRNA (shAxl279) were analyzed by flow cytometry for mouse Axl surface expression or isotype control. (B) Temporal *in vivo* imaging of bioluminescence from orthotropic (mammary fat pad) injected 4T1-GFP-Luc cells expressing either a mouse Axl-targeting shRNA (4T1-GFP-LucshmAxl2) or negative control human-specific shRNA (4T1-GFP-Luc-shAxl279) into BALB/c mice (*i*). Quantification of whole-body bioluminescence (total photon) in control (4T1-GFP-Luc shAxl279, *solid line*) and Axl-knockdown (4T1-GFP-Luc-shmAx12, *grey line*) injected BALB/c mice over an 8 week period (*ii*). *p<0.05 (t-test), *N*= 7 mice/group. (C) Survey of spontaneous metastasis (at 8-weeks post-orthotopic implantation) to different organs monitored by ex *vivo* bioluminescence detection of 4T1-GFP-Luc cells in excised organs from control or Axl-knockdown tumor bearing BALB/c mice.

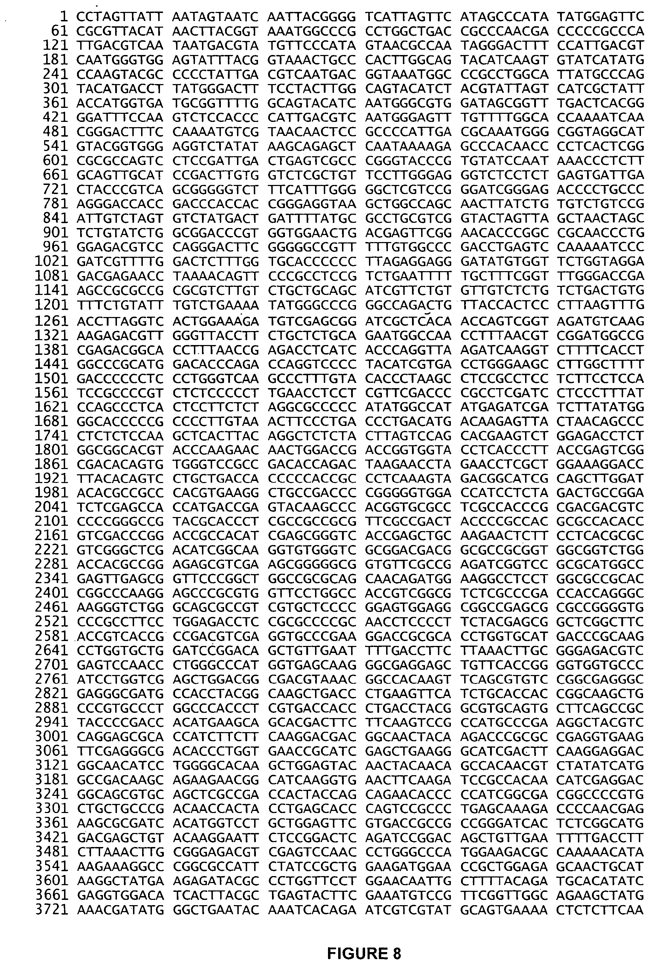

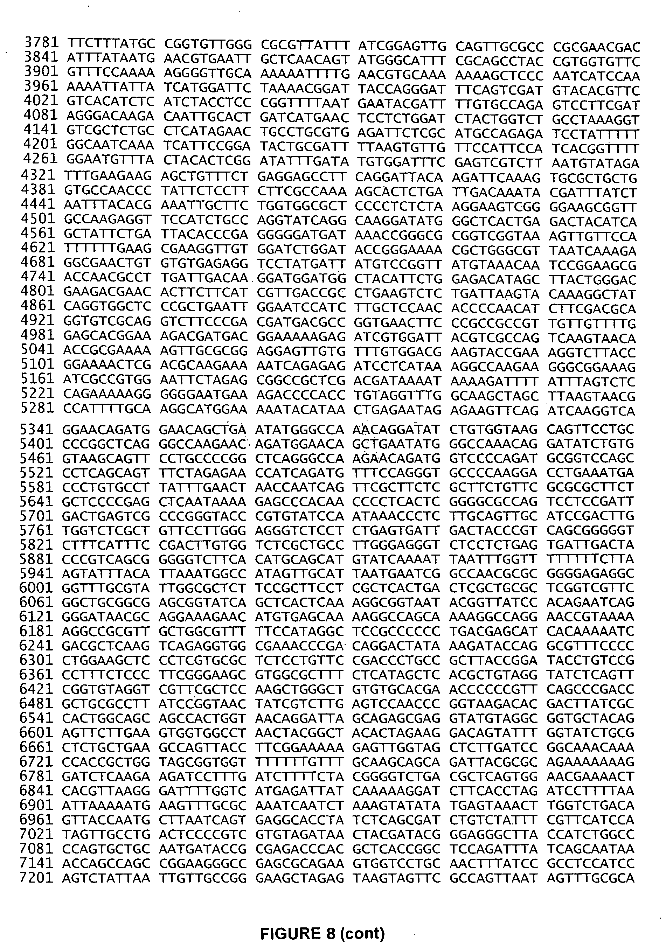

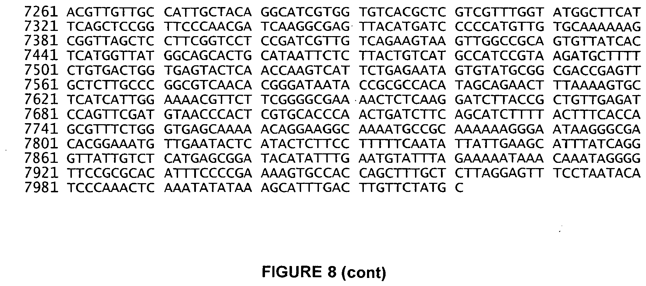

Figure 8 shows the sequence for the vector L383 pCSI Puro2AGFP2ALuc2.

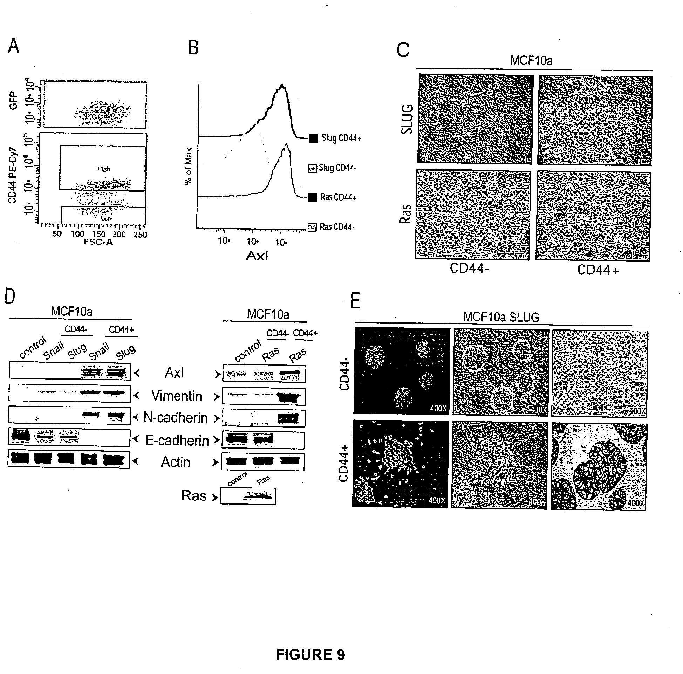

Figure 9, Panel A, shows flow cytometry analysis of MCF10a cells transduced with Slug or Ha-Ras (pBABE puro H-Ras V12, Addgene) constructs, analysed using co-expression of GFP; Ha-Ras expression was selected by puromycin treatment for 48 hours. Slug and Ha-Ras expression in MCF10a cells led to a strong increase in surface expression of the cancer stem cell marker CD44.
Figure 9, Panel B, shows that Axl surface expression in MCF10a cells encoding Lsug, Ras. Axl surface expression correlates with the presence of CD44 and mesenchymal traits both in Slug and Ha-Ras induced EMT.
Figure 9, Panel C, shows mesenchymal morphology of Slug or Ha-Ras expressing MCF10a cells at 72 hours post seeding sorted by FACS for CD44 high (CD44+) and low (CD44-) CD44-expressing sub-populations. CD44- cells show epithelial morphology, while CD44+ MCF10 cells demonstrate elongated mesenchymal morphology.
Figure 9, Panel D, shows Western blot analysis of CD44- and CD44+MCF10a cells transduced with retroviral vectors encoding Slug, Ras. CD44- MCF10a cells retained epithelial junctional and cytoskeletal protein expression. In contrast, CD44+ cells showed strong mesenchymal marker expression (vimentin, N-cadherin) and loss of E-cadherin, demonstrative of EMT.
Figure 9, Panel E, shows growth of the CD44+ and CD44- Slug and Ha-Ras expressing MCF10a cells in 3-D matrigel. CD44+, Axl-expressing MCF10a cells are invasive, consistent with a mesenchymal phenotype.

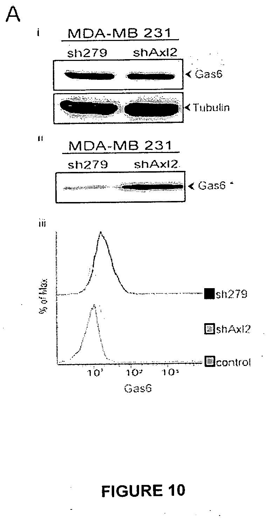

Figure 10 shows that MCF10a cells constitutively express (Top, Western blot, total lysate) and secrete (Middle, Western blot, conditioned medium) Gas6 that becomes cell-associated (Bottom, anti-Gas6, flow cytometry analysis) on Slug- and Snail-induced Axl expression.
Figure 11 shows that MDA-MB-231 cells constitutively express the Axl ligand, Gas6. (Top, Western blot, total lysate that is predominately cell-associated; Middle, Western blot, conditioned medium; Bottom, anti-Gas6 flow cytometry analysis). Axl knockdown reduces cell-associated Gas6 while increasing levels in conditioned medium (Gas6*) without affecting overall expression.

### EXAMPLES

### MATERIALS AND METHODS

### Plasmids and antibodies

All shRNAs were expressed from a modified human U6 promoter in the LTR of the retroviral vectors RRI-Red/ L087 (Genbank: EU424173) used to transform MDA-MB-231 cells via retroviral infection. RRI-Red also expresses Puro2AmRed1 resulting in puromycin resistance and red fluorescence in successfully transformed cells. All Axl cDNA nucleotides are numbered as in Genbank: BC032229.

The following sequences were used: Axl2; (hairpin in small letters)
GACATCCTCTTTCTCCTGCGAAGCCCATctggtcATGGGCTTCGCAGGAGAAAGAGG ATGTC, shAxl278;
ACGGGTCTCCTTCTTTCGCCGttggatccctggtcggatccaaCGGCGAAAGAAGGAGACC CG, shAxl279;
GCTTCAGGCGATTTCCCCGGCGttggatccctggtoggatccaaCGCCGGGGAAATCGCCT GAAGC, shAxl280;
ATGCACGCCCAGCCGCACAGCGttggatccctggtcggatccaaCGCTGTGCGGCTGGGCG TGCAT shLuc;
GATTATGTCCGGTTATGTAAACAATCCGGctggtcCCGGATTGTTTACATAACCGGAC ATAATC.
The retroviral expression vector L383 pCSI Puro2AGFP2ALuc2 (see Figure 8) was made in several stages by cloning the coding sequences of puromycin-N-acetyl-transferase, EGFP, and firefly luciferase into CRU5-retroviral expression vector (Blø *et al*., 2007). Each open-reading frame was separated from the next by a linker encoding the 2A region (XXSGLRSGQLLNFDLLKLAGDVESNPGP) from foot-and-mouth disease virus. This sequence is cleaved co-translationally resulting in the production of approximately stoichiometric amounts of each protein (Lorens 2004). Plasmids expressing hSnail, hSlug and hZEB2 were constructed by cloning the appropriate fragments from constructs BC012910, BC015895 and BC060819 (Open Biosystems) respectively into the CRU5-IRES-GFP retroviral vector (Lorens *et al* 2000). Two antibodies against human Axl were used; mouse monoclonal anti-human Axl (MAB154, R&D Systems) and goat anti-human Axl (M-20, Santa Cruz). In addition, the following antibodies were employed; rabbit anti-human pAxl (Y779, R&D), rat anti-human Snail (SN9H2, Cell Signaling), mouse anti-human Slug (L40Cb, Cell Signaling), rabbit anti-human E-cadherin (24E10, Cell Signaling), rabbit anti-human N-cadherin (ab18203, Abeam), actin, mouse anti-human b-catenin (L54E2, Cell Signaling), mouse anti-human Gas6 (R&D Systems), rabbit anti-human Twist (Twist2C1a, Abcam).

### Cell culture, retroviral transductions and cell proliferation assay

All cells were cultured at 37°C, 5% CO₂. Phoenix A cells (Dr. Gary Nolan, Stanford), MDA-MB-231 human breast epithelial carcinoma cells (American Type Culture Collection, Rockville, MD), human dermal microvascular endothelial cells (HMVEC), and pulmonary artery smooth muscle cells (PASMC) (Cambrex, Walkersville, MD, USA) were maintained as previously described (Holland 2005). The clonal cell line MDA-MB-231-DH3L2N (Xenogen Corporation, Alameda, CA, USA) was cultured in Minimum Essential Medium with Earl's Balanced Salts Solution MEM/EBSS medium supplemented with 10% FBS, 1 % nonessential amino acids, 1% L-glutamine, and 1% sodium pyruvate. Phoenix A cells were transfected using the calcium phosphate method (Swift, 1999). Approximately 30 hours after transfection, the medium was changed to growth medium for the cells to be infected, supplemented with 10% FBS. Infectious supernatant was collected -48 hours after transfection. Target cells were exposed to supernatant containing 5 mg/ml protamine sulphate over night. Infected cells were selected with(1 µg/ml puromycine. Cell proliferation assay was performed to analyze the proliferation potential of the different Axl knock down cells compared to the control cell line using MTS assay from Promega. Cells were seeded in 96-well tissue culture plates either untreated or coated with either 20µl Collagen (from Rat tail, Roche), Fibronectin (Sigma) or Matrigel (BD Biosciences). 2000 cells in 100 µl medium were seeded in triplicates in 96-well plates and assayed every 24 hours using the MTS assay from Promega according to the manufacture's instructions.

### Immunostaining, Flow cytometry and cell sorting

The MDA-MB-231 cells were trypsinated using standard procedures and washed in PBS-0.2%BSA before staining with anti-Axl (#MAB154, R&D Systems) at a final concentration of 5 mg/ml in PBS-0,2 % BSA for 40 minutes at room temperature. Cells were washed twice in PBS-0.2% BSA) and incubated with secondary antibody (Goat anti-mouse APC (Allophyocyanin, crosslinked, Molecular Probes) at a final concentration of 0.2 mg/ml for 30 minutes at room temperature in the dark. Cells were washed twice with PBS-2%BSA and resuspended in 300 ml PBS-0.2% BSA before analysis on a FacsCalibur Flow Cytometer (BD Biosciences). Data analyses were carried out using the FlowJo software (Tree Star, Inc., Ashland, OR, USA).

Cells expressing high levels of GFP, RFP and low Axl (shRNA) were isolated by FACS Aria SORP with laser 488nm, 532nm, 638nm and 407nm to establish stable, homogenous populations of cells.

### Protein extracts, SDS-PAGE, immunoblotting and immunoprecipitation

Cells were lysed in RIPA buffer (PBS with 1 % (v/v) Nonidet P-40 (NP-40), 0.5% (w/v) sodium deoxycholate, 0.1% (w/v) SDS) supplemented with protease inhibitor (Complete Mini, EDTA-free, Roche #13457200) and 0.2 mM PMSF. SDS-PAGE and immunoblotting were carried out according to standard procedures. For immunoprecipitation, cells were lysed in NP-40 buffer (10%glycerol, 1% NP-40, 50mM Tris pH 7,4, 0,2 M NaCl, 2,5mM MgCl2) supplemented with protease inhibitor and PhosSTOP phosphatase inhibitor cocktail (Roche). Extracts were incubated with antibody coubled to protA/G beads for 1 hour at 4oC, and the beads were washed in NP-40 buffer four times before elution.

### Invasion assay and 3D matrigel assay

The boyden chamber chemoinvasion assay (Albini *et* a*l* 2004) was carried out using Becton Dickinson (BDFalcon cell culture inserts (8 µm), Falcon multiwellTM 24 well plate, and growth factor reduced matrigel from BD. Inserts were coated on the inside with serum free medium diluted matrigel to a final concentration of 30 µg matrigel each insert. Matrigel work is handled at 4 °C until solidification for 30 minutes at 37 °C. 5 x 105 cells, resuspended in serum free cell medium with 0.1 % BSA added on top of each insert. FBS enriched cell medium functions as a chemoattractant. After 20 hours incubation in 37 °C with 5% CO2 the cells inside the chamber were removed by a cotton swab. Cells in the other side of the membrane were fixated, and then stained with DAPI. Pictures were taken using a fluorescent microscope and the cells counted using ImageJ (http://rsb.info.nih.gov/nih-imageJ, Wayne Rasband, National).

The 3D assays were modified from Sandal *et al* 2007; 30000 cells were seeded out on gel and cultures were allowed to grow for 10-12 days before they were analysed.

### Clinical samples

The present series of breast cancers was selected from the population based Norwegian Breast Cancer Screening Program (Hordaland County),which started in 1996 with two-view mammography done every 24months. Briefly, 95 invasive interval cancers occurred during the first two screening intervals (1996-2001), and these were matched by size with 95 screen-detected tumors from a total of 317 invasive tumors during the first two rounds (median diameter 15.6 and 15.7 mm, respectively. After matching, the mean tumor size for screen detected and interval cases were 25.1 and 23.1mm, respectively, and the corresponding mean age in these groups was 62 and 59 years. In addition to age and tumor diameter (by pathologic examination), basic characteristics, such as breast density, histologic type, histologic grade, lymph node metastases, and distant metastases at diagnosis were recorded. The median time from the last mammogram to the diagnosis of interval cancer was 17.1 months. Last date of follow-up was November 31, 2004, and median follow-up time (of survivors) was 72 months. During the follow-up period, 31 patients died of breast cancer.

### Immunohistochemistry

Tissue microarray slides were used in the present study. The tissue microarray technique is tissue conserving and has been validated in several studies. Immunohistochemistry was performed on 5 µm thick sections of formalin-fixed, paraffin-embedded tissues. Antigen retrieval was performed by boiling for 10 min at 750 W and 20 min at 350 W in TRS (Target Retrieval Solution; DakoCytomation, Denmark, AS) buffer, ph 6.0, in a microwave oven. A DakoCytomation Autostainer was used for staining.

The slides were incubated overnight at room temperature with a polyclonal antibody against Axl (H-124; cat. # 20741), dilution 1:200 (Santa Cruz, USA). Immunoperoxidase staining was carried out using the DakoCytomation Envision Kit (DakoCytomation, Denmark AS) with diaminobenzidin tetrachloride peroxidase as substrate prior to counterstaining with Mayer's haematoxylin (DakoCytomation, Denmark AS).

### Evaluation of staining

The staining was predominantly cytoplasmatic, although there was some concentration of staining in the cytoplasmatic membrane. Staining was recorded by a semiquantitative and subjective grading system, considering the intensity of staining and the proportion of tumor cells showing a positive reaction. All three cores from each case were evaluated. Intensity was recorded as 0 (no staining) to 3 (strong staining); the percentage of membranous staining area was recorded as 0 (no tumor cells positive), 1 (<10%), 2 (10%-50%), and 3 (>50% of tumor cells). A staining index (SI) was calculated as the product of staining intensity and area. Immunohistochemical registration was done blinded for patient characteristics and outcome.

### Statistics

Comparisons of groups were performed by Pearson χ2 test. In all statistical analyses, cut-off values for staining index (SI) categories were based on median values. Univariate survival analyses (using death from endometrial carcinoma as end point; death from other causes were censored) were performed using the product-limit procedure (Kaplan-Meier method), with the time of primary operation as the entry date.

The log-rank (Mantel-Cox) test was used to compare survival curves for different categories of each variable. Variables with impact on survival in univariate analyses (P ≤ .15) were examined by log-log plot to determine how these variables could be incorporated in Cox' proportional hazards regression models.

### Mouse strain and animal care

In the present study we used female mice of PrkdcSCID/B2mnull (abbreviated as NOD/SCID/B2mnull), severe combined immunodeficient mice (GADES Institute, Norway), aged 8-10 weeks and weighting between 20-25g. They were kept under standard conditions in a 12h light/dark cycle and allowed at least 7 days to acclimatize to their new environmental condition prior to onset of experiment. This investigation, designed to minimize the number of animals and suffering, was carried out in accordance with the Norwegian Regulation on Animal Experimentation, the European Convention for the Protection of Vertebrate Animals used for scientific purposes and the guidelines of the Norwegian Animal Research Authority.

### Bioluminescence imaging (BLI)

BLI was performed using a eXplore Optix (GE Healtcare) camera mounted in a specimen box. Imaging and quantification of signals was done using eXplore Optix software. For *in vivo* imaging, animals received via intraperitoneal injection (i.p) the substrate D-luciferin (Biosynth), 150mg/kg in PBS (Phosphate Buffered Saline) and anesthetized with isoflurane. Mice were placed into warmed stage inside of camera box with continuous exposure to 1-2% isoflurane and imaged for different views depending on the tumor model. Region of interest were identified and were quantified as total photon/sec-1 using eXplore Optix software (GE Explore Optix). *In vivo* background bioluminescence was in the range of 2-3×10 photon counts. For ex *vivo* imaging, 150 mg/kg D-luciferin was injected into the mice just before necropsy. Tissues of interest were excised, placed into plates and imaged.

### In vivo tumor models

### Tissue engineering

1x106 MDA-MB 231 cells which express GFP-Luc biomarker and different RNAs interference that regulate Axl expression, were suspended in a 1:1 mixture of F-12 Kaighn's (Invitrogen) :Matrigel (BD Biosciences) and seeded in 6x6 mm Poly-lactic acid (PLLA) scaffolds.

Females NOD/SCID/B2mnull were anesthetized by exposure to 1-2% isoflurane (Isoba vet.-Schering-Plough A/S) during the implantation procedure and on subsequent imaging days. Two scaffolds were implanted subcutaneously (s.c) in each mouse according to Nor et al. Lab Invest.; 81 (4) 453; 2001 Scaffolds with cells that express Axl were implanted on the left side of each mouse while on the right side scaffolds with cells in which Axl was knockdown. After the surgical procedure, anesthetized mice were placed in Imaging System and imaged for both left and right sides 10-15 min after intraperitoneal injection of 150mg/kg D-luciferin (Biosynth). Tumor development was monitored *in vivo* once a week by imaging for 4 weeks.

### Xenograft assay

1 x 106 MDA-MB-231 cells infected with shRNA vectors and GFP-Luc were suspended in 100µl of F12K medium + 10% FBS/ Matrigel (1:1 ratio, BD Biosciences) and injected with a 29-gauge insulin needle subcutaneously into both flanks of female NOD/SCID/B2m. At the left flank positive cells on Axl expression were injected, while in right flank cells negative in Axl expression were injected.

For tricellular implant MDA-MB-231 cells infected with shRNA vectors and GFP-Luc were mixed with Human dermal microvascular endothelial cells (HMVEC), and pulmonary artery smooth muscle cells (PASMC) in ratio of 1:2:2. Tumor growth was monitored weekly *in vivo* by imaging for 4 consecutive weeks.

### Mammary fat pad spontaneous metastasis model

Subline of human MDA-MB-231 cells (called MDA-MB 231 DH3L2N - Xenogen) which express GFP-Luc biomarker and different RNAs interference that regulate Axl expression were injected into mammary pad of mice. NOD/SCID/B2mnull mice were anesthetized by exposure to 1-3% isoflurane and injected with 50 µl of 2 × 106 MDA-MB-231 DH3L2N cells suspended in MEM/EBSS medium/Matrigel (1:1) into the abdominal mammary fat pad. 10-15 min after D-luciferin (Biosynth) injection, mice were placed in the eXplore Optix Imaging System and imaged from the ventral view. Tumor growth and metastasis spread was monitored every second week by bioluminescent imaging for up to 9 weeks. The lower part of each animal was covered before reimaging, to minimize the bioluminescence from the primary tumor so that the signals from the metastatic regions could be observed *in vivo.*

### Tissue collection

At the end of each experiment the tumor tissues implants and different organs were retrieved from the mice and preserved in 10% Paraformaldehyde (Sigma-Aldrich) for further analysis. Tissues was prepared for histopathology (paraffin embedding, sectioning and staining) and analyzed by microscope evaluation.

### Statistical analysis of animal model results

The mean bioluminescence (photons/sec-1), tumor diameter and corresponding standard errors were determined for each experiment. Regression plots were used to describe the relationship between bioluminescence, cell number and tumor diameter. Statistical analyses were based on paired t-test.

### RESULTS

### Axl expression is a strong prognostic factor for overall survival of breast cancer patients

In order to assess the role of Axl in breast cancer pathogenesis, we investigated Axl expression in tumors from a series of breast cancer patients identified during a Norwegian Breast Cancer Screening Program which started in 1996 entailing bi-annual two-view mammography (Wang *et al* 2001). Briefly, 95 invasive interval cancers occurred during the first two screening intervals (1996-2001), and these were matched by size with 95 screen-detected tumors from a total of 317 invasive tumors during the first two rounds (median diameter 15.6 and 15.7 mm, respectively (Collett *et al.,* 2005). After matching, the mean tumor size for screen detected and interval cases were 25.1 and 23.1mm, respectively, and the corresponding mean age in these groups was 62 and 59 years. In addition to age and tumor diameter (by pathologic examination), basic characteristics, such as breast density, histologic type, histologic grade, lymph node metastases, and distant metastases at diagnosis were recorded. The median time from the last mammogram to the diagnosis of interval cancer was 17.1 months. Clinical parameters were monitored, last date of follow-up was November 31, 2004, and median follow-up time (of survivors) was 72 months. During the follow-up period, 31 patients died of breast cancer.

There were no significant associations between Axl expression (divided in two groups by median staining index; Figure 1A) and important clinico-pathologic features such as histologic grade, tumor diameter, expression of estrogen and progesterone receptors, and axillary lymph node status. Also, Axl expression was not associated with HER2, E-cadherin, markers of basal differentiation (Cytokeratin 5/6, P-cadherin), EZH2, or tumor cell proliferation by Ki-67 expression.

However, univariate survival analysis (Kaplan-Meier method, log-rank test), Axl expression was significantly associated with reduced patient survival (p=0.035; Figure 1B). In multivariate analysis (proportional hazards method), including basic prognostic factors like tumor diameter, histologic grade and lymph node status in addition to Axl expression (step one), Axl expression status remained as an independent negative prognostic factor in the final model (p=0.021), in addition to histologic grade and lymph node status (see Figure 1C). Thus, Axl expression is a strong prognosticator of poor clinical outcome in breast cancer patients.

We then investigated Axl expression in patient biopsies of matched pairs (n=16) of primary and metastatic breast carcinomas. Axl expression tended to be further elevated in metastases when compared with corresponding primary human breast carcinomas (p=0.11, McNemar's test; Figure 1d; metastases to the right, liver (*upper*) and bone (*lower*)), suggesting that Axl expression is a strong prognosticator of poor clinical outcome in breast cancer patients and associated with metastatic spread.

### Axl is required for breast cancer cell invasiveness

The strong correlation of Axl expression in early breast carcinomas with poor survival indicates an important role for Axl in overall disease pathogenesis. As breast cancer-related mortality invariably results from complications of metastatic disease, we assessed whether Axl expression was required for malignant breast carcinoma cell invasiveness. Axl is expressed in several highly metastatic human breast carcinoma cell lines including MB-MDA-231. The Axl ligand, Gas6 is often co-expressed, leading to autocrine activation (Holland *et al.*, 2005). In order to effectively correlate Axl expression levels in MB-MDA-231 cells with specific cellular behaviors, we developed an epi-allelic series of Axi-targeting shRNAs that reduce Axl expression in a dose dependent manner, using a recently developed FACS-based RNAi approach, CellSelectRNAi (Figure 2A; Micklem *et al*., in preparation). This Axl shRNA collection was used to create an epi-allelic Axl MB-MDA-231 cell series with graded total (Figure 2B) and phosphorylated (Figure 2C) Axl protein levels.

Malignant carcinoma cells exhibit mesenchymal cell invasiveness in three-dimensional extracellular matrix protein gels (Matrigel) that correlates with *in vivo* metastatic potential (Bissell). Epi-allelic analysis demonstrated a dose-dependent requirement for Axl expression for MB-MDA-231 cell invasion in response to serum (Figure 2D) or the SDF-1 chemokine (Figure 2E), an important factor in breast carcinoma metastasis. In contrast, Axl knockdown had no effect on MB-MDA-231 cell proliferation and only a modest effect on two-dimensional (lateral epithelial wound healing) migration. This indicated a specific requirement for Axl in three-dimensional growth and invasiveness. We therefore evaluated the effect of Axl knockdown on MB-MDA-231 cells in a 3D-Matrigel assay. Normal breast epithelial cells self-organize into polarized spheroid acinar structures in 3D-Matrigel, while malignant MB-MDA-231 cells proliferate forming large disorganized colonies with invasive, stellate outgrowths that reflect aggressive tumors (Bissell). Knockdown of Axl expression strongly reversed the malignant phenotype of MB-MDA-231 cells in 3D-Matrigel, creating small round colonies without malignant outgrowths (Figure 2F,G). Together, these data suggest that Axl signaling is required to maintain the mesenchymal-like invasiveness of metastatic breast carcinoma cells.

### Axl is upregulated by EMT-inducing transcription factors in breast epithelial cells

The acquisition of mesenchymal invasiveness, the ability to migrate and invade ECM, is the functional hallmark of EMT. The EMT-inducing transcription factor Twist is required for metastasis of breast carcinoma cells (Yang *et al* 2004). We therefore investigated if Twist expression upregulates Axl in breast epithelial cells. Twist expression in normal breast epithelial cells (MCF10A) induces EMT (Figure 3A; Glackin *et al*., in preparation). Strikingly, Axl is also strongly upregulated in Twist-expressing MCF10A cells (Figure 3A-B). Further, as Gas6 is constitutively expressed by normal breast epithelial cells, this Twist-induced Axl expression establishes an autocrine activation loop, evidenced by increased cell-associated Gas6 and tyrosine phosphorylated Axl (Figure 3A, C). To determine if other EMT inducing transcription factors similarly upregulate Axl expression, we analyzed MCF10A cells expressing ZEB2, Snail and Slug. Each of these EMT transcription factors induced mesenchymal transition in MCF10A cells and upregulated Axl expression. These results suggest that EMT induction leads to Axl expression and can establish autocrine signaling.

### Axl expression is necessary for tumor formation in experimental tissue engineered breast tumors

In order to evaluate the requirement for Axl for malignant growth *in vivo* in we used a tissue engineering approach comprising MB-MDA-231 cells that express a GFPluciferase construct for efficient *in vivo* optical imaging (CSI; Tiron et *al*., unpublished results), seeded with Matrigel into poly-lactic acid tissue engineering scaffolds and implanted subcutaneously into immunocompromised NOD-SCID mice, Growth within engineered tumor microenviroments is associated with tumor cell mesenchymal characteristics as tumor cells colonize the scaffold (Mooney). MB-MDA-231 cells readily form tumors this biomimetic microenviroment, displaying aggressive colonization of the scaffold (Figure 4A). Axl knockdown strongly inhibited tumor formation, lateral spread and malignant morphology (Figure 4A).

To ascertain whether Axl influences the ability of breast cancer cells to attract and co-opt blood vessels, we developed a tri-cellular implant approach comprising MB-MDA-231 cells seeded together with primary human microvascular endothelial (HuMVEC) and vascular smooth muscle cells (vSMC) to create tumor vasculature. Implants of human EC-vSMC cells readily form perfused intrascaffold human microvasculature in NOD-SCID mice within a two-week period (Hegen *et al*., in preparation). As shown in Figure 4B, MB-MDA-231 cells form aggressive, highly vascularized tumors in this tri-cellular implant model. The engineered human tumor vasculature is evenly distributed and perfused with intralumenal red blood cells. In contrast, Axl knockdown, blocked tumor formation, without affecting development of a perfused human microvasculature. This indicates that Axl is required for tumor formation even in the presence of a microvasculature that likely obviates the need for induced angiogenesis.

### A distinct Axl expression threshold is required for breast tumor formation

In order to evaluate the level of Axl expression needed to form a tumor, we conducted an *in vivo* epi-alletic analysis of Axl in subcutaneous MDA-MB-231 tumors. The Axl epi-allelic MDA-MB-231 cell series (Figure 1) was injected subcutaneously and temporally monitored for tumor formation by bioluminescent scanning. This approach revealed an Axl dose response for tumor growth (Figure 5). Correlation with surface Axl levels demonstrated a threshold of Axl expression required for tumor formation (Figure 5B,C). This dose response is congruent with the dose dependent effects of Axl inhibition on invasiveness (Figure 2).

### Axl is essential for metastasis of breast carcinoma cells

In order to evaluate the requirement of Axl for breast cancer metastasis, we orthotopically injected MDA-MB-231-D3H2LN, a rapidly growing and highly metastatic *in vivo* MDA-231 isolate, into the mammary fat pad. Using whole body bioluminescent imaging we temporally monitored spontaneous metastasis development over a 9-week period. Control MDA-MB-231-D3H2LN cells generated large orthotopic mammary tumors that became necrosing within 5-6 weeks (Figure 6A). Axl knockdown in MDA-MB-231-D3H2LN reduced the rate of primary mammary tumor formation but also grew substantial primary mammary tumors (Figure 6A). Spontaneous metastasis was initially detected in the thoracic sentinel lymph node of control MDA-MB-231-D3H2LN injected mice at 4 weeks (Figure 6A). In contrast no metastases were detected in MDA-231DHLN-AxlshRNA implanted mice. Upon sacrifice at 9 weeks, excised organs were scanned individually for bioluminescence due to the presence of metastatic cells. MDA-MB-231-D3H2LN injected mice had formed extensive spontaneous metastasis in all mice, including lymph node, lung, ovaries and kidneys. In contrast, MDA-MB-231 DHLN-AxlshRNA cells did not form detectable metastasis (apart from a single lesion in the kidney of one mouse). Histological analysis of tissue biopsies from the organs of MDA-MB-231-D3H2LN injected mice confirmed the presence of multiple micro- and macrometastases in all organs as predicted by the bioluminescent total photon measurements (Figure 6C). No micro- or macrometastases were observed in tissue biopsies from MDA-MB-231 DHLN-AxlshRNA injected mice, confirming that the lack of observed bioluminescence was due to inhibited metastasis formation. These results show that Axl is essential for breast carcinoma metastasis.

We evaluated the functional contribution of Axl to overall survival of NOD-SCID mice with orthotopically injected MDA-MB-231-D3H2LN/GFP-Luc control or Axl knockdown cells. Overall survival was significantly increased in MDA-MB-231-D3H2LN/GFP-Luc-shAxl2 tumor-bearing mice (P=0.013, log-rank test; Figure 6d). These results together with our clinical observations support the conclusion that Axl is an important to the development of metastatic disease and overall patient survival.

In order to validate our results in a different metastatic model we transduced the highly metastatic mouse breast carcinoma 4T1 cell line with the CSI-construct and selected for GFPluciferase expression by FACS (4T1-GFP-Luc). The 4T1 cells are dependent on Twist expression for metastasis and exhibit high levels of Axl expression (Figure 7a).

We developed a retroviral vector that expresses a mouse Axl-targeting shRNA (shmAxl2) that effectively suppresses mouse Axl surface levels in 4T1 cells (Figure 7a). A mismatched human Axl-targeting shRNA (shAxl279) had no effect on mouse Axl expression. Similar to Twist knockdown in 4T1 cells and results with MDA-MB-231 cells, tissue culture expansion of the Axl-knockdown 4T1 cells was not significantly affected (data not shown).

When introduced into the mammary gland of female normal BALB/c mice, the syngenic 4T1 tumor cells display a biphasic growth pattern, due to a rigorous immune response that leads to tumor regression associated with leukocyte infiltration and necrosis, followed by re-growth at the primary site that coincides with extensive metastasis to multiple organs. 4T1-GFPLuc cells show only monophasic growth in immunocomprimised NOD-SCID mice (data not shown). We injected 4T1-GFP-Luc cells expressing either a mouse Axl-targeting shRNA (4T1-GFP-Luc-shmAxl2) or negative control human-specific shRNA (4T1-GFP-LucshAxl279) into the mammary fat pad of female BALB/c mice and quantified tumor growth and metastasis by temporal whole-body in vivo optical imaging (Figure 7b). The control 4T1-GFP-Luc-shmAxl2 cells displayed rapid primary growth, reaching a maximum after one week. This was followed by a precipitous regression that was sustained for five weeks (Figure 7b). At week 6, recurrence at the primary site and multiple distant metastases were observed that subsequently grew rapidly, causing moribundity and lethality in all mice by week 8. The 4T1-GFP-Luc cells expressing the mouse Axl-targeting shRNA (4T1-GFP-Luc-shmAxl2) initially followed a similar course: a rapid primary tumor growth, slightly attenuated by Axlsuppression, followed by regression (Figure 7b). However, the subsequent recurrence of the primary tumor and emergence of rapidly growing distant metastasis was completely absent (Figure 7b). Indeed, all mice injected with the 4T1-GFP-Luc-shmAxl2 cells remained healthy at the time of sacrifice (8 weeks). The splenomegaly associated with the leukemoid reaction characteristic of the 4T1 model was also reduced in mice bearing Axl-knockdown tumors (data not shown). Upon sacrifice at 8 weeks, individual excised organs were imaged and total light emission quantified, confirming the presence of metastases at common dissemination sites in all mice bearing 4T1-GFP-Luc-shAxl279 tumors (Figures 7c-d). In contrast, no bioluminescent tumor cells were detected in the organs from mice with 4T1-GFP-LucshmAxl2 tumors (Figures 7d-e). These results support the conclusion that Axl is an essential regulator of breast tumor metastasis.

### Autocrine Regulation by Gas6

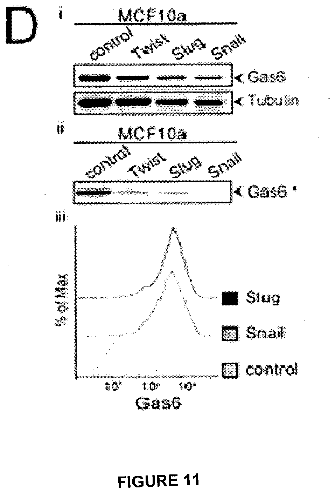

The Axl ligand Gas6 is often coexpressed with Axl, consistent with autocrine activation. Cell-associated Gas6 and phosporylated Axl levels indicative of autocrine signaling are reduced upon Axl knockdown in MDA-MB-231 cells (Figure 11). In MCF10A cells, Gas6 was constitutively expressed and became cell associated on EMT-induced Axl expression (Figure 11). These results suggest that EMT program induction leads to Axl expression and can establish autocrine signaling in breast epithelial cells. As Axl, often coexpressed with Gas6, is detected in many metastatic cancers, autocrine Axl signaling may be a frequent consequence of EMT in many tumor types. EMT-inducing transcription factors such as Snail, Slug, and Twis potently induce Axl expression, suggesting that Axl could participate in a positive feedback loop that sustains the malignant mesenchymal phenotype of tumor cells. This notion is consistent with our observation of elevated Axl expression in metastatic lesions.

### CD44+ Phenotype is associated with Axl Expression

MCF10a cells were transduced with Slug or Ha-Ras (pBABE puro H-Ras V12, Addgene) constructs. Slug transduced cells were analysed by flow cytometry using coexpression of GFP; Ha-Ras expression was selected by puromycin treatment for 48 hours. As shown by flow cytometry (Figure 9, panel A), Slug and Ha-Ras expression in MCF10a cells led to a strong increase in expression surface expression of the cancer stem cell marker CD44. Flow cytometry analysis of MCF10a cells transduced with Slug or Ha-Ras further showed a strong increase in surface Axl expression. Slug or Ha-Ras expressing MCF10a cells were sorted by FACS for CD44 high (CD44+) and low (CD44-) CD44-expressing sub-populations. The CD44- cells showed epithelial morphology while the CD44+ MCF10 cells demonstrated elongated mesenchymal morphology (Figure 9, Panel C). Western blot analysis of these cells (Figure 9, Panel D) demonstrated that CD44- MCF10a cells retained epithelial junctional and cytoskeletal protein expression. In contrast, CD44+ cells showed strong mesenchymal marker expression (vimentin, N-cadherin) and loss of E-cadherin, demonstrative of EMT. Axl expression correlated with the presence of CD44 and mesenchymal traits both in Slug and Ha-Ras induced EMT (Figure 9, Panels B, D). Growth of the CD44+ and CD44- Slug and Ha-Ras expressing MCF10a cells in 3-D matrigel (Figure 9, Panel E) demonstrated that the CD44+, Axl-expressing MCF10a cells are invasive, consistent with a mesenchymal phenotype. These results demonstrate that Axl is upregulated by Slug and Ha-Ras expression in MCF10a cells and correlates with mesenchymal and cancer stem cell traits.

### REFERENCES

Camp, R.L., V. Neumeister, and D.L. Rimm, A Decade of Tissue Microarrays: Progress in the Discovery and Validation of Cancer Biomarkers. J Clin Oncol, 2008.
Collett, K., et al., A basal epithelial phenotype is more frequent in interval breast cancers compared with screen detected tumors. Cancer Epidemiol Biomarkers
Elston, C.W. and I.O. Ellis, Pathological prognostic factors in breast cancer. I. The value of histological grade in breast cancer: experience from a large study with long-term follow-up. Histopathology, 1991. 19(5): p. 403-10.Prev, 2005. 14(5): p. 1108-12.
Gupta PB, Mani S, Yang J, Hartwell K, Weinberg RA. The evolving portrait of cancer metastasis.Cold Spring Harb Symp Quant Biol. 2005;70:291-7.
Holland S. J., Friera A.M., Franci C., Chan E., Atchison R., McLaughlin J., Swift S. E., Pali E., Yam G., Wong S., Lasaga J., Shen M., Yu S., Xu W., Hitoshi Y., Payan D.G, Nor J. E., Powell M.J, and Lorens J.B. (2005) The Receptor Tyrosine Kinase Axl Regulates Angiogenesis and Tumor Growth. Cancer Research 65:9294-9303.
Kononen, J., et al., Tissue microarrays for high-throughput molecular profiling of tumor specimens. Nat Med, 1998. 4(7): p. 844-7.
Sun, W., Fujimoto, J. & Tamaya, T. Coexpression of Gas6/Axl in human ovarian cancers. Oncology 66, 450-7 (2004).
Thiery JP. Epithelial-mesenchymal transitions in tumour progression. Nat Rev Cancer. 2002 Jun;2(6):442-54
Vajkoczy P, Knyazev P, Kunkel A, Capelle HH, Behrndt S, von Tengg-Kobligk H, Kiessling F, Eichelsbacher U, Essig M, Read TA, Erber R, Ullrich A Dominant-negative inhibition of the Axl receptor tyrosine kinase suppresses brain tumor cell growth and invasion and prolongs survival. Proc Natl Acad Sci U S A. (2006)103:5799-804.
Yang J, Mani SA, Weinberg RA Exploring a new twist on tumor metastasis. Cancer Res 2006 66:4549-52.
Yang J, Mani SA, Donaher JL, Ramaswamy S, Itzykson RA, Come C, Savagner P, Gitelman I, Richardson A, Weinberg RA.Twist, a master regulator of morphogenesis, plays an essential role in tumor metastasis. Cell. 2004 117:927-39.
Yang J, Weinberg RA. Epithelial-mesenchymal transition: at the crossroads of development and tumor metastasis.Dev Cell. 2008 Jun;14(6):818-29.
Wang, H., et al., Mammography screening in Norway: results from the first screening round in four counties and cost-effectiveness of a modeled nationwide screening. Cancer Causes Control, 2001. 12(1): p. 39-45.
Holland et al. R428, a selective small molecule inhibitor of Axl kinase, blocks tumour spread and prolongs survival in models of metastatic cancer. Cancer Research; 70(4), February 15, 2010.

## Claims

1. Use of Axl as a biomarker for detecting the occurrence of epithelial-to- mesenchymal transition (EMT) in a subject.

2. Use according to claim 1 wherein Axl is a biomarker for detecting the induction of epithelial-to-mesenchymal transition (EMT).

3. Use according to claim 1 or claim 2 wherein upregulation of Axl is indicative of the occurrence of epithelial-to-mesenchymal transition (EMT).

4. A method for detecting the occurrence of epithelial-to-mesenchymal transition (EMT) in a sample, said method comprising
determining the expression of Axl in a sample isolated from a cell, group of cells, an animal model or human as compared to a control sample, wherein upregulation of Axl expression relative to the control sample is indicative of the occurrence of epithelial-to-mesenchymal transition (EMT).

5. A method for identifying an agent capable of inhibiting or reversing epithelial-to-mesenchymal transition (EMT), said method comprising administering said agent to a cell, group of cells or animal model, not a human, and monitoring the activity and/or the expression of Axl.

6. A method according to claim 5 which comprises:
(i) administering the agent to a cell, group of cells or an animal model, not a human; and
(ii) measuring Axl expression in samples derived from the treated and the untreated cells or animal model; and
(iii) detecting an increase or a decrease in the expression of Axl in the treated sample as compared to the untreated sample as an indication of the ability to inhibit or reverse epithelial-to-mesenchymal transition (EMT).

7. A method according to claim 6 wherein the sample is analysed by protein analysis.

8. A method according to claim 7 wherein protein analysis is by ELISA, PET, flow cytometry, SELDI-TOF MS or 2-D PAGE.

9. A method according to any one of claims 4 to 8, wherein the group of cells is a cell culture.

10. A method according to any one of claims 4 to 9, wherein the cells are tumor cells, PBMC or lymphocytes.

11. A method according to claim 10, wherein the tumour cells are metastatic cells in lymphatic or haematogenous circulation.

12. A method according to any one of claims 4 or 6 to 10 wherein the sample is blood.

13. A method according to any one of claims 4 or 6 to 10 wherein the sample is serum, plasma or tissue culture supernatant.

14. A method according to claim 5 which comprises the steps of:
(i) contacting the agent with Axl receptor tyrosine kinase or cells expressing the Axl receptor tyrosine kinase;
(ii) measuring the Axl receptor tyrosine kinase activity in the presence of the agent; and
(iii) comparing the activity measured in step (ii) to that measured under controlled conditions, wherein a decrease identifies the agent as being capable of inhibiting or reversing epithelial-to-mesenchymal transition (EMT).

15. A method according to claim 14 wherein the activity measured is tyrosine phosphorylation of a substrate of the Axl receptor tyrosine kinase.

16. A method according to claim 14 wherein the activity measured is auto phosphorylation of the Axl receptor tyrosine kinase.

17. A method according to claim 14 wherein the cells in the contacting step (i) have previously been transfected by the Axl gene.

18. A method according to claim 17 wherein the transfected cells are either transiently or stably transfected.

19. A method according to claim 14 wherein the controlled conditions in step (iii) comprises contacting the agent with cells that lack an active Axl gene.

20. A method according to claim 19 wherein the cells have a mutated inactive form of the Axl gene.

21. A method according to claim 14 wherein the Axl receptor tyrosine kinase comprises a biologically active portion of the intracellular domain.

22. A method according to claim 15 wherein the Axl receptor tyrosine kinase is immobilized.

23. A method according to claim 5 which comprises screening a plurality of agents, said method comprising the steps of:
(i) contacting the plurality of agents with the Axl receptor tyrosine kinase or cells expressing the Axl receptor tyrosine kinase with;
(ii) measuring the Axl receptor tyrosine kinase activity in the presence of the plurality of agents;
(iii) comparing the activity measured in step (ii) to that measured under controlled conditions, wherein a decrease identifies the plurality of agents as being capable of inhibiting or reversing epithelial-to-mesenchymal transition (EMT);
and
(v) separately determining which agent or agents present in the plurality inhibit or reverse epithelial-to-mesenchymal transition (EMT).

24. Use of a kit for assessing the ability of an agent to inhibit or reverse epithelial- to-mesenchymal transition (EMT), said kit comprising anti-Axl antibodies.

25. Use of a kit for assessing the ability of an agent to inhibit or reverse epithelial- to-mesenchymal transition (EMT), said kit comprising a nucleic acid probe for Axl.

26. Use of a kit for assessing the ability of an agent to inhibit or reverse epithelial- to-mesenchymal transition (EMT), said kit comprising at least one QPCR primer for Axl.

27. Use of a kit as defined in any of claims 24 to 26 in a method defined in any of claims 5 to 22.

## Patentansprüche

1. Verwendung von Axl als Biomarker zum Detektieren des Auftretens von epithelial-mesenchymaler Transition (EMT) bei einem Individuum.

2. Verwendung nach Anspruch 1, wobei Axl ein Biomarker zum Detektieren der Induktion von epithelial-mesenchymaler Transition (EMT) ist.

3. Verwendung nach Anspruch 1 oder Anspruch 2, wobei eine Hochregulierung von Axl das Auftreten von epithelial-mesenchymaler Transition (EMT) anzeigt.

4. Verfahren zum Detektieren des Auftretens von epithelial-mesenchymaler Transition (EMT) in einer Probe, wobei das Verfahren Folgendes umfasst:
das Bestimmen der Expression von Axl in einer Probe, die von einer Zelle, Gruppe von Zellen, einem Tiermodell oder einem Menschen isoliert wurde, im Vergleich zu einer Kontrollprobe, wobei eine Hochregulierung der Axl-Expression im Vergleich zur Kontrollprobe das Auftreten von epithelial-mesenchymaler Transition (EMT) anzeigt.

5. Verfahren zur Identifikation eines Mittels, das in der Lage ist, epithelial-mesenchymale Transition (EMT) zu hemmen oder umzukehren, wobei das Verfahren das Verabreichen des Mittels an eine Zelle, Gruppe von Zellen oder Tiermodell, nicht aber an einen Menschen, und das Überwachen der Aktivität und/oder Expression von Axl umfasst.

6. Verfahren nach Anspruch 5, das Folgendes umfasst:
(i) das Verabreichen des Mittels an eine Zelle, Gruppe von Zellen oder ein Tiermodell, nicht aber an einen Menschen; und
(ii) das Messen der Axl-Expression in von den behandelten und den nicht behandelten Zellen oder Tiermodell stammenden Proben; und
(iii) das Detektieren einer Zunahme oder einer Abnahme der Expression von Axl in der behandelten Probe im Vergleich zur unbehandelten Probe als Indikation der Fähigkeit, epithelial-mesenchymale Transition (EMT) zu hemmen oder umzukehren.

7. Verfahren nach Anspruch 6, wobei die Probe mittels Proteinanalyse analysiert wird.

8. Verfahren nach Anspruch 7, wobei die Proteinanalyse mittels ELISA, PET, Durchflusszytometrie, SELDI-TOF-MS oder 2-D-PAGE erfolgt.

9. Verfahren nach einem der Ansprüche 4 bis 8, wobei die Gruppe von Zellen eine Zellkultur ist.

10. Verfahren nach einem der Ansprüche 4 bis 9, wobei die Zellen Tumorzellen, PBMC oder Lymphozyten sind.

11. Verfahren nach Anspruch 10, wobei die Tumorzellen metastatische Zellen im Lymphsystem oder im Blutkreislauf sind.

12. Verfahren nach einem der Ansprüche 4 oder 6 bis 10, wobei die Probe Blut ist.

13. Verfahren nach einem der Ansprüche 4 oder 6 bis 10, wobei die Probe Serum, Plasma oder Gewebekulturüberstand ist.

14. Verfahren nach Anspruch 5, das die folgenden Schritte umfasst:
(i) das Inkontaktbringen des Mittels mit Axl-Rezeptor-Tyrosinkinase oder Zellen, die die Axl-Rezeptor-Tyrosinkinase exprimieren;
(ii) das Messen der Axl-Rezeptor-Tyrosinkinaseaktivität in Gegenwart des Mittels; und
(iii) das Vergleichen der in Schritt (ii) gemessenen Aktivität mit jener, die unter kontrollierten Bedingungen gemessen wurde, wobei eine Abnahme das Mittel als fähig zur Hemmung oder Umkehrung von epithelial-mesenchymaler Transition (EMT) identifiziert.

15. Verfahren nach Anspruch 14, wobei die gemessene Aktivität die Tyrosinphosphorylierung eines Substrats der Axl-Rezeptor-Tyrosinkinase ist.

16. Verfahren nach Anspruch 14, wobei die gemessene Aktivität die Autophosphorylierung der Axl-Rezeptor-Tyrosinkinase ist.

17. Verfahren nach Anspruch 14, wobei die Zellen im Schritt des Inkontaktbringens (i) davor mit dem Axl-Gen transfiziert wurden.

18. Verfahren nach Anspruch 17, wobei die transfizierten Zellen entweder transient oder stabil transfiziert werden.

19. Verfahren nach Anspruch 14, wobei die kontrollierten Bedingungen aus Schritt (iii) das Inkontaktbringen des Mittels mit Zellen umfassen, denen ein aktives Axl-Gen fehlt.

20. Verfahren nach Anspruch 19, wobei die Zellen eine mutierte inaktive Form des Axl-Gens aufweisen.

21. Verfahren nach Anspruch 14, wobei die Axl-Rezeptor-Tyrosinkinase einen biologisch aktiven Teil der intrazellulären Domäne umfasst.

22. Verfahren nach Anspruch 15, wobei die Axl-Rezeptor-Tyrosinkinase immobilisiert ist.

23. Verfahren nach Anspruch 5, welches das Screening einer Vielzahl von Mitteln umfasst, wobei das Verfahren die folgenden Schritte umfasst:
(i) das Inkontaktbringen der Vielzahl von Mitteln mit der Axl-Rezeptor-Tyrosinkinase oder Zellen, die die Axl-Rezeptor-Tyrosinkinase exprimieren;
(ii) das Messen der Axl-Rezeptor-Tyrosinkinase-Aktivität in Gegenwart der Vielzahl von Mitteln;
(iii) das Vergleichen der in Schritt (ii) gemessenen Aktivität mit jener, die unter kontrollierten Bedingungen gemessen wurde, wobei eine Abnahme die Vielzahl von Mitteln als fähig zur Hemmung oder Umkehrung von epithelial-mesenchymaler Transition (EMT) identifiziert; und
(v) das separate Bestimmen, welches oder welche der in der Vielzahl enthaltenen Mittel epithelial-mesenchymale Transition (EMT) hemmen oder umkehren.

24. Verwendung eines Sets zur Bestimmung der Fähigkeit eines Mittels zur Hemmung oder Umkehrung von epithelial-mesenchymaler Transition (EMT), wobei das Set Anti-Axl-Antikörper umfasst.

25. Verwendung eines Sets zur Bestimmung der Fähigkeit eines Mittels zur Hemmung oder Umkehrung von epithelial-mesenchymaler Transition (EMT), wobei das Set eine Nucleinsäuresonde für Axl umfasst.

26. Verwendung eines Sets zur Bestimmung der Fähigkeit eines Mittels zur Hemmung oder Umkehrung von epithelial-mesenchymaler Transition (EMT), wobei das Set zumindest einen QPCR-Primer für Axl umfasst.

27. Verwendung eines Sets wie in einem der Ansprüche 24 bis 26 definiert in einem Verfahren nach einem der Ansprüche 5 bis 22.

## Revendications

1. Utilisation d'Axl en tant que biomarqueur pour détecter l'occurrence d'une transition épithélio-mésenchymateuse (TEM) chez un sujet.

2. Utilisation selon la revendication 1, dans laquelle Axl est un biomarqueur pour détecter l'induction d'une transition épithélio-mésenchymateuse (TEM).

3. Utilisation selon la revendication 1 ou la revendication 2, dans laquelle la régulation positive d'Axl est indicative de l'occurrence d'une transition épithélio-mésenchymateuse (TEM).

4. Procédé pour détecter l'occurrence d'une transition épithélio-mésenchymateuse (TEM) dans un échantillon, ledit procédé comprenant
la détermination de l'expression d'Axl dans un échantillon isolé à partir d'une cellule, d'un groupe de cellules, d'un modèle animal ou d'un humain par comparaison à un échantillon de contrôle, dans lequel une régulation positive de l'expression d'Axl par rapport à l'échantillon de contrôle est indicative de l'occurrence d'une transition épithélio-mésenchymateuse (TEM).

5. Procédé pour identifier un agent capable d'inhiber ou d'inverser une transition épithélio-mésenchymateuse (TEM), ledit procédé comprenant l'administration dudit agent à une cellule, un groupe de cellules ou un modèle animal, non un humain, et la surveillance de l'activité et/ou de l'expression d'Axl.

6. Procédé selon la revendication 5, qui comprend :
(i) l'administration de l'agent à une cellule, un groupe de cellules ou un modèle animal, non un humain ; et
(ii) la mesure de l'expression d'Axl dans des échantillons dérivés des cellules ou du modèle animal traité(es) et non traité(es) ; et
(iii) la détection d'une augmentation ou d'une réduction de l'expression d'Axl dans l'échantillon traité par comparaison à l'échantillon non traité en tant qu'indication de la capacité à inhiber ou inverser une transition épithélio-mésenchymateuse (TEM).

7. Procédé selon la revendication 6, dans lequel l'échantillon est analysé par une analyse protéique.

8. Procédé selon la revendication 7, dans lequel l'analyse protéique est réalisée par ELISA, TEP, cytométrie en flux, MS SELDI-TOF ou PAGE 2-D.

9. Procédé selon l'une quelconque des revendications 4 à 8, dans lequel le groupe de cellules est une culture cellulaire.

10. Procédé selon l'une quelconque des revendications 4 à 9, dans lequel les cellules sont des cellules tumorales, des PBMC ou des lymphocytes.

11. Procédé selon la revendication 10, dans lequel les cellules tumorales sont des cellules métastatiques dans la circulation lymphatique ou hématogène.

12. Procédé selon l'une quelconque des revendications 4 ou 6 à 10, dans lequel l'échantillon est du sang.

13. Procédé selon l'une quelconque des revendications 4 ou 6 à 10, dans lequel l'échantillon est du sérum, du plasma ou un surnageant de culture tissulaire.

14. Procédé selon la revendication 5, qui comprend les étapes consistant à :
(i) mettre en contact l'agent avec un récepteur tyrosine kinase Axl ou des cellules exprimant le récepteur tyrosine kinase Axl ;
(ii) mesurer l'activité du récepteur tyrosine kinase Axl en présence de l'agent ; et
(iii) comparer l'activité mesurée à l'étape (ii) à celle mesurée dans des conditions contrôlées, où une réduction identifie l'agent comme étant capable d'inhiber ou d'inverser une transition épithélio-mésenchymateuse (TEM).

15. Procédé selon la revendication 14, dans lequel l'activité mesurée est la phosphorylation sur tyrosine d'un substrat du récepteur tyrosine kinase Axl.

16. Procédé selon la revendication 14, dans lequel l'activité mesurée est l'auto-phosphorylation du récepteur tyrosine kinase Axl.

17. Procédé selon la revendication 14, dans lequel les cellules, à l'étape de mise en contact (i), ont précédemment été transfectées par le gène Axl.

18. Procédé selon la revendication 17, dans lequel les cellules transfectées sont transfectées de manière transitoire ou stable.

19. Procédé selon la revendication 14, dans lequel les conditions contrôlées à l'étape (iii) comprennent la mise en contact de l'agent avec des cellules qui sont dépourvues de gène Axl actif.

20. Procédé selon la revendication 19, dans lequel les cellules comportent une forme inactive mutée du gène Axl.

21. Procédé selon la revendication 14, dans lequel le récepteur tyrosine kinase Axl comprend une partie biologiquement active du domaine intracellulaire.

22. Procédé selon la revendication 15, dans le récepteur tyrosine kinase Axl est immobilisé.

23. Procédé selon la revendication 5, qui comprend le criblage d'une pluralité d'agents, ledit procédé comprenant les étapes consistant à :
(i) mettre en contact la pluralité d'agents avec le récepteur tyrosine kinase Axl ou des cellules exprimant le récepteur tyrosine kinase Axl ;
(ii) mesurer l'activité du récepteur tyrosine kinase Axl en présence de la pluralité d'agents ;
(iii) comparer l'activité mesurée à l'étape (ii) à celle mesurée dans des conditions contrôlées, où une réduction identifie la pluralité d'agents comme étant capable d'inhiber ou d'inverser une transition épithélio-mésenchymateuse (TEM) ; et
(iv) séparément déterminer quel(s) agent ou agents présent(s) dans la pluralité inhibe(nt) ou inverse(nt) une transition épithélio-mésenchymateuse (TEM).

24. Utilisation d'un kit pour évaluer la capacité d'un agent à inhiber ou inverser une transition épithélio-mésenchymateuse (TEM), ledit kit comprenant des anticorps anti-Axl.

25. Utilisation d'un kit pour évaluer la capacité d'un agent à inhiber ou inverser une transition épithélio-mésenchymateuse (TEM), ledit kit comprenant une sonde d'acide nucléique pour Axl.

26. Utilisation d'un kit pour évaluer la capacité d'un agent à inhiber ou inverser une transition épithélio-mésenchymateuse (TEM), ledit kit comprenant au moins une amorce de QPCR pour Axl.

27. Utilisation d'un kit tel que défini dans l'une quelconque des revendications 24 à 26 dans un procédé défini dans l'une quelconque des revendications 5 à 22.
